# EUROPEAN PATENT APPLICATION

(11) **EP 4 316 396 A1**
(43) Date of publication of application: **07.02.2024**
(21) Application number: 22778779.3
(22) Date of filing: 25.03.2022
(51) Int. Cl.: A61B 17/34, A61M 25/00

(54) **PUNCTURE DEVICE**

(30) Priority: 29.03.2021 CN 202110336027; 11.05.2021 CN 202110508588; 11.05.2021 CN 202110508562
(71) Applicant: Shenzhen Lifetech Respiration Scientific Co., Ltd., Shenzhen, Guangdong 518000 (CN)
(72) Inventor: LI, Anning, Shenzhen, Guangdong 518000 (CN); QIU, Libiao, Shenzhen, Guangdong 518000 (CN)
(74) Representative: Prinz & Partner mbB
(86) International application number: PCT/CN2022/083027
(87) International publication number: WO 2022/206596

(57) **Abstract**

A puncture device, including a handle assembly and a puncture assembly that is received in the handle assembly and fixedly connected to the handle assembly, where the puncture assembly includes a puncture member; the handle assembly further includes a first control element for controlling the puncture member; and when the first control element rotates, the first control element controls the puncture member to move close to or away from the handle assembly. The first control element controls the puncture member to move close to or away from the handle assembly to facilitate the first control element to control the puncture member to pass out of the handle assembly, which is easy to operate, and it is convenient for controlling the puncture member to carry out puncture surgery. The puncture member can be prevented from continuously moving and harming the body due to inertia after a successful puncture during puncture surgery.

## Description

### Technical Field

The embodiments relate to the field of medical instruments and to a puncture device.

### Background

A traditional puncture needle is made of a stainless steel tube. In a puncture surgery with a traditional puncture member, a doctor usually holds a connector of the puncture member with hands and applies a thrust to the connector of the puncture member to make a needle tip of the puncture member puncture the atrial septum. Because of the inertial effect, after the doctor punctures the atrial septum with the puncture member, the doctor continues to push the connector of the puncture member with the hand, so that the needle tip of the puncture member continues to move, resulting in an excessive travel. In this way, the left atrial wall is easily stabbed, leading to pericardial tamponade. Or, the aorta is easily punctured, which causes serious complications.

### Summary

Based on this, it is necessary to provide a puncture device, which avoids that a puncture member easily continues to move after puncturing the atrial septum in puncturing surgery.

A puncture device includes a handle assembly and a puncture assembly received in the handle assembly and fixedly connected to the handle assembly; the puncture assembly includes a puncture member; the handle assembly further includes a first control element used for controlling the puncture member; and when the first control element rotates, the first control element controls the puncture member to move close to or away from the handle assembly.

The first control element controls the puncture member to move close or away from the handle assembly, so that it is convenient for the first control element to control the puncture member to pass out of the handle assembly, which is easy to operate and convenient for controlling the puncture member to carry out puncture surgery. The puncture member can be prevented from continuously moving and harming the body due to inertia after a successful puncture during the traditional puncture surgery.

### Brief Description of the Drawings

Fig. 1 is a schematic structural diagram of a puncture device provided by a first embodiment.
Fig. 2 is an exploded diagram of a puncture device provided by a first embodiment.
Fig. 3 is a schematic diagram of a puncture assembly in the first embodiment in a receiving state.
Fig. 4a is a schematic structural diagram of a top view of a handle body in the first embodiment.
Fig. 4b is a schematic diagram of a first handle piece, a first control element and a second control element.
Fig. 4c is a schematic diagram of a second handle piece, a first control element and a second control element.
Fig. 5 is a schematic diagram of a puncture assembly in the first embodiment in an extending state.
Fig. 6a is a schematic diagram of connection of a first fixing member, a second fixing member, an outer tube and a puncture member.
Fig. 6b is a schematic structural diagram of a first fixing member.
Fig. 7 is a schematic structural diagram of part of the second control element.
Fig. 8a is a schematic structural diagram of a second fixing member.
Fig. 8b is a schematic structural diagram of a top view of a second fixing member.
Fig. 8c is a schematic structural diagram of a sealing member.
Fig. 9 is a schematic diagram of bending and deformation of a puncture assembly in the first embodiment.
Fig. 10 is a schematic structural diagram of a puncture member in the first embodiment.
Fig. 11a is a schematic diagram of a supporting portion and a main body portion in a second embodiment.
Fig. 11b is a schematic diagram of a distal end of a puncture assembly in the second embodiment.
Fig. 11c is an enlarged schematic structural diagram of position X in Fig. 11b.
Fig. 12 is a schematic diagram of a bending adjustment member in the second embodiment.
Fig. 13 is a schematic diagram of a bending adjustment member in the third embodiment.
Fig. 14 is a schematic structural diagram of a puncture device provided by a fourth embodiment.
Fig. 15 is a schematic sectional structural diagram of Fig. 14.
Fig. 16 is an enlarged schematic structural diagram of figure X in Fig. 15.
Fig. 17 is a schematic sectional structural diagram of Fig. 14 in another angle.
Fig. 18 is a schematic exploded diagram in the fourth embodiment.
Fig. 19 is a schematic structural diagram of a first control element in the fourth embodiment.
Fig. 20 is a schematic structural diagram of an anti-miscontact member in the fourth embodiment.
Fig. 21 is a schematic sectional structural diagram of an anti-miscontact member in a fifth embodiment.
FIG 22 is a schematic enlarged diagram of position XX in FIG 21.
FIG 23 is a schematic enlarged diagram of position XX in another embodiment.

### Detailed Description of the Embodiments

In order to make the foregoing objectives, features and advantages of the embodiments more clear and understandable, the implementation modes of the embodiments are described in detail with reference to the accompanying drawings. Many specific details are described in the following descriptions to facilitate full understanding of the embodiments. However, the embodiments can be implemented in a variety of other ways than those described herein, and those skilled in the art can make similar improvements without departing from the connotation of the embodiments. Therefore, the embodiments are not limited by the implementations below.

Unless otherwise defined, all terms used herein are the same as meanings of general understandings of those skilled in the art of the embodiments. The terms used in the description herein are merely to describe the specific embodiments, not intended to limit the embodiments.

In the field of interventional medical apparatuses, "distal end" is defined as an end far from an operator during surgery, and "proximal end" is defined as an end close to the operator during surgery. "Axial direction" refers to a direction parallel to a connecting line between a center of a distal end of a medical apparatus and a center of a proximal end, and "radial direction" refers to a direction perpendicular to the above axial direction.

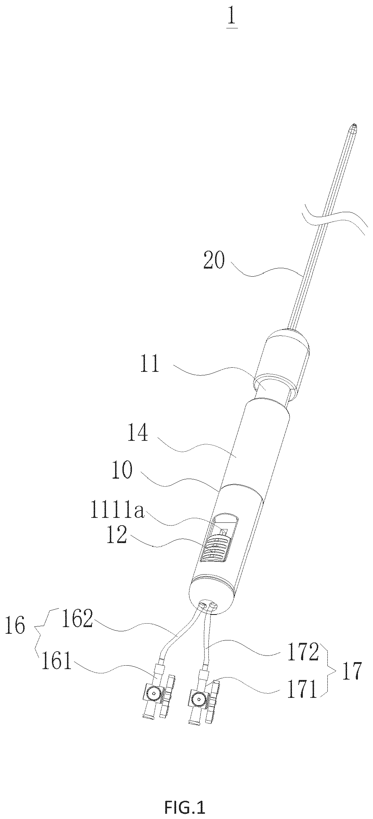

Referring to Fig. 1 to Fig. 5, a first embodiment provides a puncture device 1. The puncture device 1 is used for performing puncturing in a human body, for example, but not limited to, fossa ovalis puncturing, atrial septum puncturing, or puncturing in transjugular intrahepatic portacaval shunt. In this embodiment, the puncture device 1 punctures the fossa ovalis, which is taken for example for description.

Referring to Fig. 1 to Fig. 3, the puncture device 1 includes a handle assembly 10, and a puncture assembly 20 received in the handle assembly 10 and fixedly connected to the handle assembly 10.

The puncture assembly 20 includes an outer tube 23 and a puncture member 24. The outer tube 23 includes an outer tube body 21. The puncture member 24 includes a puncture needle 22. The outer tube body 21 is connected with the handle assembly 10. The outer tube body 21 is a hollow tube. In this embodiment, the puncture needle 22 is inserted with the outer tube body 21. At least part of the puncture needle 22 is received in the outer tube body 21. In this embodiment, an extending direction of the puncture needle 22 in the outer tube body 21 is the same as that of the outer tube body 21. A distal end 22a of the puncture needle 22 is close to a distal end 21a of the outer tube body 21. A proximal end 22b of the puncture needle 22 passes out of a proximal end 21b of the outer tube body 21.

In this embodiment, at least part of the puncture needle 22 is received in the outer tube body 21, and the distal end 22a of the puncture needle 22 is close to the distal end 21a of the outer tube body. The puncture needle 22 moves into the body together with the outer tube body 21. During the puncture surgery, the puncture needle 22 does not need to be inserted from the proximal end 21b of the outer tube body 21 first, and then slide to the distal end 21a of the outer tube body 21, so as to avoid damage to an inner wall of the outer tube body 21 during the sliding of the puncture needle 22 in the outer tube body 21 and production of debris, so that the risk of thrombosis caused by the debris to harm patients is lowered.

In this embodiment, the distal end 22a of the puncture needle 22 is close to the distal end 21a of the outer tube body 21, which means that the distal end 22a of the puncture needle 22 is close to an end face of the distal end 21a of the outer tube body 21, and an end face of the distal end 22a of the puncture needle 22 is not flush with an end face of the distal end 21a of the outer tube body 21, that is, the puncture needle 22 is received in the outer tube body 21 or part of the puncture needle 22 is exposed from the outer tube body 21.

In this embodiment, when the outer tube body 21 slides to a designated area of the body, the distal end 22a of the puncture needle 22 is received in the outer tube body 21 without being exposed from the outer tube body 21. For example, when the outer tube body 21 slides near the fossa ovalis of the heart, the outer tube body 21 would bend and deform when it meets a curved blood vessel channel of the body, while the puncture needle 22 is not exposed from the outer tube body 21, so as to prevent the puncture needle 22 from stabbing the body.

In this embodiment, the puncture needle 22 runs through the outer tube body 21, and the proximal end 22b of the puncture needle 22 is also close to the proximal end 21b of the outer tube body 21. For example, the proximal end 22b of the puncture needle 22 extends out of the proximal end 21b of the outer tube body 21.

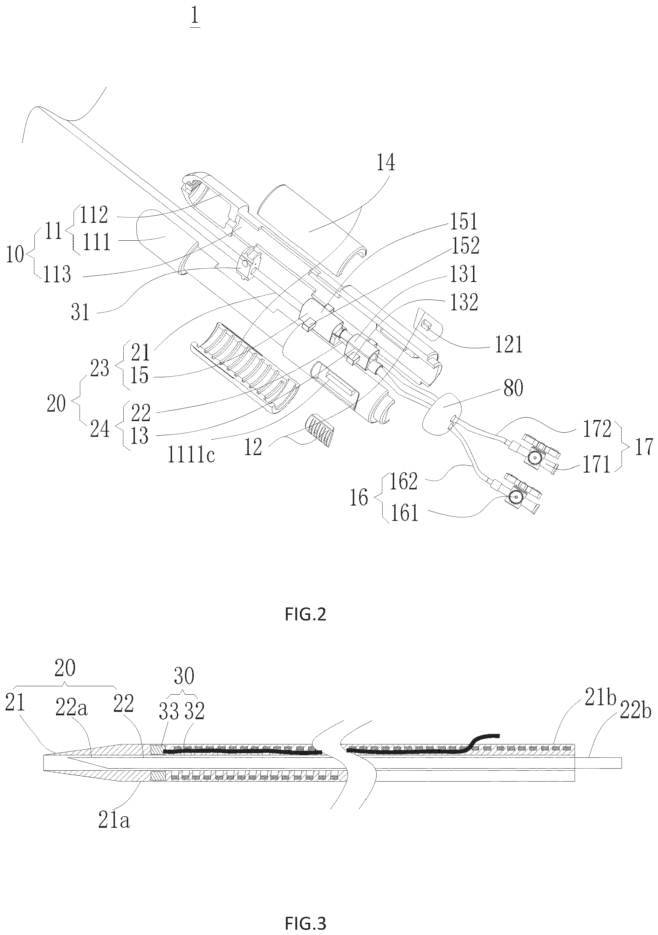

Referring to Fig. 1 and Fig. 2, in this embodiment, the handle assembly 10 has a receiving member 113. The receiving member 113 receives part of the puncture assembly 20. The receiving member 113 receives the proximal end of the puncture needle 22 and the proximal end of the outer tube body 21. For example, the handle assembly 10 includes a handle body 11. The handle body 11 includes a first handle piece 111 and a second handle piece 112 connected with the first handle piece 111. In this embodiment, a receiving space enclosed by the first handle piece 111 and the second handle piece 112 is the receiving member 113.

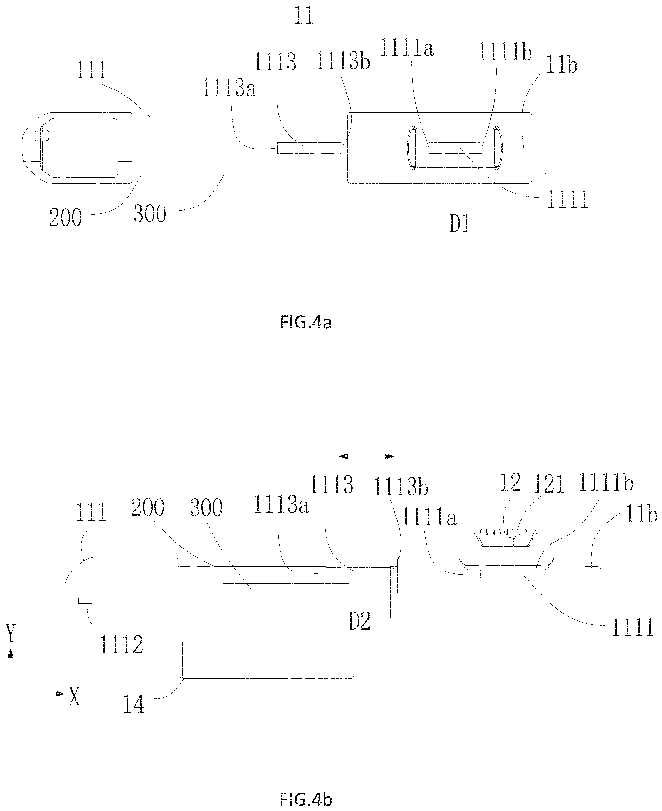

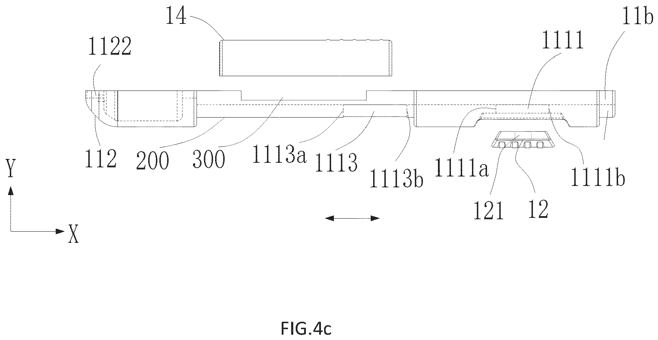

Referring to Fig. 4a, Fig. 4b and Fig. 4c, the first handle piece 111 is provided with a fastening piece 1112. The second handle piece 112 is provided with an abutment member 1122. In this embodiment, the fastening piece 1112 protrudes from the first handle piece 111 axially towards the handle assembly 10, and the fastening piece 1112 is hook-shaped as a whole. The fastening piece 1112 protrudes towards a negative direction of a Y-axis. The abutment member 1122 is recessed from the second handle piece 112 away from the handle assembly 10, and the abutment member 1122 is groove-shaped as a whole. In Fig. 4c, the abutment member 1122 is recessed towards the negative direction of the Y-axis. The abutment member 1122 and the fastening piece 1112 cooperate with each other to make the first handle piece 111 and the second handle piece 112 closed and fixed together. It can be understood that in other embodiments, the fastening piece 1112 can be a groove, and the abutment member 1122 can be a boss.

In this embodiment, the first handle piece 111 is fixedly connected with the second handle piece 112. For example, glue (not shown in the figure) is also provided between the first handle piece 111 and the second handle piece 112. The glue fixedly connects the first handle piece 111 with the second handle piece 112. In other embodiments, by other means, such as high-temperature melting, or by providing a thread on an outer wall of the first handle piece 111 and also providing a thread on an outer wall of the second handle piece 112, the first handle piece 111 and the second handle piece 112 cooperate with each other and are in threaded connection with a locking member with an internal thread. The specific method is not limited, as long as it can fix the first handle piece 111 and the second handle piece 112.

It can be understood that in other embodiments, the handle assembly 10 may be integrally formed. That is, the first handle piece 111 and the second handle piece 112 are integrally formed.

Referring to Fig. 1, Fig. 3 and Fig. 5, in this embodiment, the puncture assembly 20 can slide relative to the handle assembly 10 in the axial direction. For example, the puncture needle 22 in the puncture assembly 20 can slide relative to the handle body 11 in the handle assembly 10 in the axial direction. The puncture assembly 20 has a receiving state and an extending state. The receiving state and the extending state can be interchanged. The extending state refers to that part of the distal end 22a of the puncture needle 22 is exposed from the distal end 21a of the outer tube body 21. The receiving state means that the distal end 22a of the puncture needle 22 is received within the distal end 21a of the outer tube body 21.

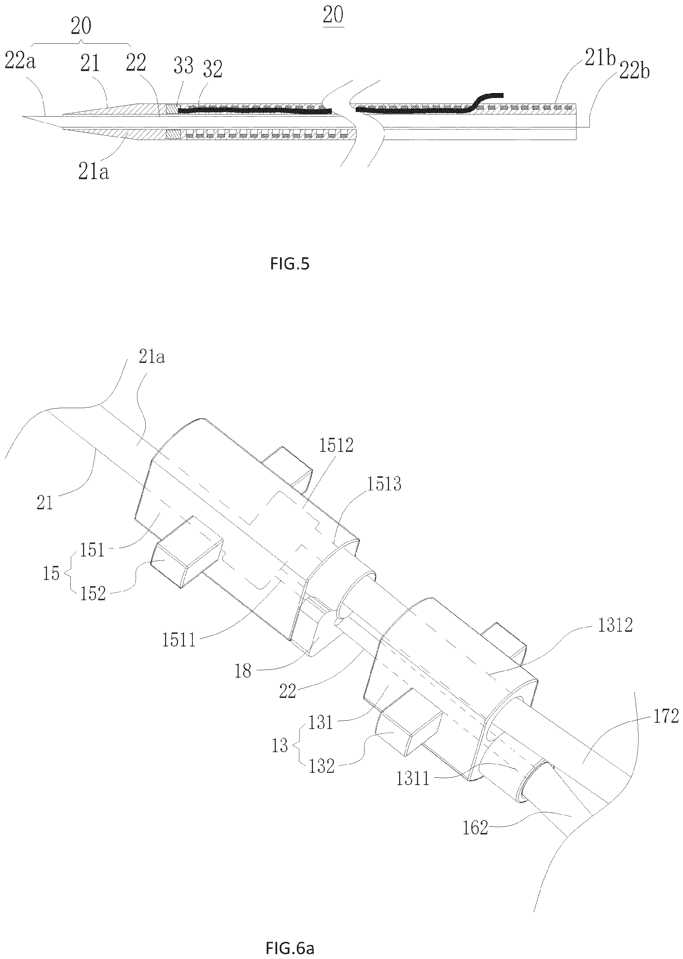

The handle assembly 10 also includes a first control element 12. The first control element 12 is provided on at least one of the first handle piece 111 and the second handle piece 112. Referring to Fig. 1 and Fig. 2, in this embodiment, there are two first control elements 12 which are respectively arranged on the first handle piece 111 and the second handle piece 112. The first control elements 12 are connected with the proximal end of the puncture needle 22. The first control elements 12 are used for controlling the puncture needle 22 to axially slide relative to the handle body 11. That is, referring to Fig. 5, the first control element (not shown in the figure) can control the puncture needle 22 to slide axially, so that the distal end 22a of the puncture needle 22 extends out of the outer tube body 21. Or, referring to Fig. 3, the first control element controls the distal end 22a of puncture needle 22 to extend into the outer tube body 21.

It can be understood that after receiving an external acting force, the first control element 12 transmits the acting force to the puncture needle 22 to make the puncture needle 22 slide axially. In this embodiment, the first control element 12 receives external action to slide axially relative to the handle body 11. The first control element 12 is connected with the puncture needle 22, and the first control element 12 slides with the puncture needle 22 to make the puncture needle 22 pass out of the outer tube body 21 for the puncture surgery. It can be understood that in this embodiment, the first control element 12 is indirectly connected with the proximal end of the puncture needle 22. In other embodiments, the first control element 12 can be directly connected with the puncture needle 22.

At least one of the first handle piece 111 and the second handle piece 112 is provided with a first limiting hole 1111. The first limiting hole 1111 restrains a travel of the first control element 12. The first limiting hole 1111 is communicated with the receiving member 113. Referring to Fig. 2, Fig. 4a, Fig. 4b and Fig. 4c, in this embodiment, the first handle piece 111 and the second handle piece 112 are both provided with first limiting holes 1111. The first limiting hole 1111 in the first handle piece 111 and the first limiting hole 1111 in the second handle piece 112 have the same shape, and the first limiting hole 1111 in the first handle piece 111 directly faces the first limiting hole 1111 in the second handle piece 112. In this embodiment, the first limiting hole 1111 is a through hole. It can be understood that in other embodiments, there can be only one first control element 12 and one first limiting hole 1111 used cooperatively with the first control element 12.

Referring to Fig. 2, in this embodiment, the puncture member 24 also includes the first fixing member 13. The first fixing member 13 is received in the receiving member 113. The first fixing member 13 is connected with the proximal end (not shown in the figure) of the puncture needle 22. For example, the first fixing member 13 includes a first fixing member body 131. The proximal end of the puncture needle 22 extends into the first fixing member body 131 and is fixedly connected with the first fixing member body 131. It can be understood that at least part of the first fixing member body 131 directly faces the first limiting hole 1111. That is, at least part of a projection of the first limiting hole 1111 in a radial direction falls on the first fixing member body 131. In this embodiment, the first control element 12 receives the external action to slide axially relative to the handle body 11, and the first control element 12 drives the first fixing member 13 and the puncture needle 22 to slide axially relative to the handle body 11. In other embodiments, an external force directly acts on the first fixing member 13 to make the first fixing member 13 slide axially relative to the handle body 11, and the first fixing member 13 drives the puncture needle 22 to slide axially relative to the handle body 11.

Referring to Fig. 2, in this embodiment, the first fixing member 13 also includes a first fixing portion 132 connected with the first fixing member body 131. The first fixing portion 132 is connected with an outer wall of the first fixing member body 131. In this embodiment, the first fixing portion 132 is integrated with the first fixing member body 131. The first fixing portion 132 is connected with the first control element 12 through the first limiting hole 1111. For example, the first fixing portion 132 is arranged on the outer wall of the first fixing member 13 and protrudes radially outwards from the outer wall of the first fixing member 13. Part of the first fixing portion 132 passes through the first limiting hole 1111 and is then connected with the first control element 12. It can be understood that part of the first fixing portion 132 directly faces the first limiting hole 1111. That is, part of the first fixing portion 132 directly faces a hole wall of the first limiting hole 1111.

In this embodiment, a portion of the first fixing portion 132 passing through the first limiting hole 1111 is fixedly connected with the first control element 12. For example, the first control element 12 is provided with a first clamping portion 121. The first fixing portion 132 extends into the first clamping portion 121 and is fixedly connected with the first clamping portion 121. The first clamping portion 121 is a groove. The first fixing portion 132 is in interference fit with the first clamping portion 121. In other embodiments, the first clamping portion 121 and the first fixing portion 132 are also bonded with glue to stably connect the first clamping portion 121 with the first fixing portion 132.

In this embodiment, the number of the first fixing portion 132, the number of the first limiting hole 1111 and the number of the first control element 12 are the same. That is, there are two first fixing portions 132 which respectively pass through the first limiting hole 1111 of the first handle piece 111 and the first limiting hole 1111 of the second handle piece 112. The two first fixing portions 132 are symmetrically distributed about the axial direction of the handle assembly 10, so that the first fixing member 13 is uniformly stressed as a whole, and the first fixing member 13 can stably slide in the axial direction relative to the handle body 11. In other embodiments, the number of the first fixing portion 132 is not limited, as long as the first control element 12 can control the puncture needle 22 to slide through the first fixing portion 132. In other embodiments, the first fixing portion 132 is a groove, which is inserted with the first clamping portion 121 and fixed in the first fixing portion 132.

Referring to Fig. 4a, Fig. 4b and Fig. 4c, in this embodiment, the first limiting hole 1111 includes a first limiting portion 1111a and a second limiting portion 1111b opposite to the first limiting portion 1111a. The first limiting portion 1111a and the second limiting portion 1111b are arranged at intervals in the axial direction. The first limiting portion 1111a and the second limiting portion 1111b are two opposite hole walls of the first limiting hole 1111 respectively. Other hole walls of the first limiting hole 1111 form the first limiting hole 1111 with the first limiting portion 1111a and the second limiting portion 1111b. The first limiting portion 1111a is a distal end of the first limiting hole 1111, and the second limiting portion 1111b is a proximal end of the first limiting hole 1111.

The first limiting hole 1111 restrains the axial travel of the first control element 12. That is, the first limiting portion 1111a and the second limiting portion 1111b restrain the axial travel of the first control element 12 to restrain the axial travels of the first fixing member 13 and the puncture needle 22. The axial sliding process of the puncture needle 22 is as follows.

Referring to Fig. 2, Fig. 4b, Fig. 4c and Fig. 5, on the one hand, the first control element 12 slides from the proximal end of the first limiting hole 1111 towards the distal end of the first limiting hole 1111. The first control element 12 drives the first fixing portion 132 and the puncture needle 22 to slide away from the proximal end 11b of the handle body 11, and the puncture needle 22 gradually extends out of the outer tube body 21. Part of the first fixing portion 132 directly facing the first limiting hole 1111 is restrained by the distal end of the first limiting hole 1111 after abutting against the distal end of the first limiting hole 1111. The first fixing portion 132 cannot continue to slide away from the proximal end 11b of the handle body 11. The puncture needle 22 and the first control element 12 cannot continue to slide away from the proximal end 11b of the handle body 11, either.

On the other hand, the first control element 12 slides from the distal end of the first limiting hole 1111 towards the proximal end of the first limiting hole 1111. The first control element 12 drives the first fixing portion 132 and the puncture needle 22 to slide towards the proximal end 11b of the handle body 11. Part of the first fixing portion 132 directly facing the first limiting hole 1111 is restrained by the second limiting portion 11 11b after abutting against the proximal end of the first limiting hole 1111. The first fixing portion 132 cannot slide towards the proximal end 11b of the handle body 11. The puncture needle 22 and the first control element 12 cannot continue to slide towards the proximal end of the handle body 11.

In this embodiment, a cross-sectional area of the first fixing member body 131 is larger than that of the first limiting hole 1111, so that the first fixing member body 131 cannot pass through the first limiting hole 1111, making the first fixing member 13 not easy to leave the handle body 11.

An axial distance D1 between the first limiting portion 1111a and the second limiting portion 1111b corresponds to the travel of the entire puncture needle 22 in one puncture process. The travel of the first control element 12 sliding in the first limiting hole 1111 away from the proximal end of the handle body 11 is the same as the sliding travel of the entire puncture needle 22. Since the puncture needle 22 bends in the body, the travel of the distal end of the puncture needle 22 is less than or equal to the travel of the entire puncture needle 22, and is less than or equal to an axial distance between the first limiting portion 1111a and the second limiting portion 11 11b.

In a puncture surgery with a traditional puncture member, a doctor usually holds a connector of the puncture member with hands and applies a thrust to the connector of the puncture member to make a needle tip of the puncture member puncture the atrial septum. Because of the inertial effect, after the doctor punctures the atrial septum with the puncture member, the doctor continues to push the connector of the puncture member with the hand, so that the needle tip of the puncture member continues to move, resulting in an excessive travel. In this way, the left atrial wall is easily stabbed, leading to pericardial tamponade. Or, the aorta is easily punctured, which causes serious complications. Furthermore, a thrust applied by the doctor to the connector of the puncture member is not uniform, which will affect the sliding of the puncture member in a sheath tube. After penetrating through the atrial septum, the puncture needle 22 is restrained by the first limiting hole 1111 from continuing to move, so as to prevent the puncture needle 22 from continuing to stabbing the left atrial wall, thus improving the safety of the puncture surgery with the puncture needle 22. Moreover, the two first control elements 12 are respectively arranged on the first handle piece 111 and the second handle piece 112. The acting force applied by the doctor is transmitted to the puncture needle 22 by pushing the two first control elements 12, making the acting force on the puncture needle 22 more uniform. The puncture needle 22 can axially slide smoothly, which improves the puncture stability of the puncture needle 22.

Referring to Fig. 1, Fig. 4b and Fig. 4c, in this embodiment, an outer wall of the first control element 12 is provided with a plurality of bulges arranged at intervals, and a protruding direction of the bulges is opposite to an opening direction of the first clamping portion 121. These bulges can increase the friction force between doctor's hand and the first control element 12, so that the doctor can push the first control element 12 to slide.

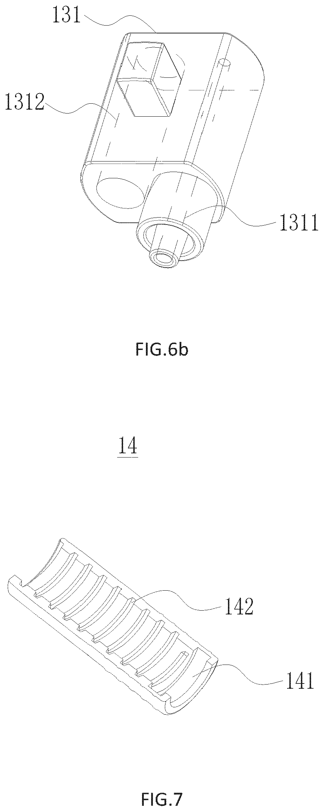

Referring to Fig. 2, Fig. 6a and Fig. 6b again, the first fixing member body 131 is provided with a first connecting member channel 1311. The proximal end (not shown in the figure) of the puncture needle 22 is fixed in the first connecting member channel 1311, and an inner cavity of the puncture needle 22 is communicated with the first connecting member channel 1311.

The puncture device 1 also includes a first connecting member 16. The first connecting member channel 1311 is communicated with the inside of the first connecting member 16 and the inside of the puncture needle 22, so that a first liquid medium can be delivered into the body through the first connecting member 16, the first connecting member channel 1311 and the puncture needle 22 in sequence. In this embodiment, the puncture needle 22 is communicated with the first connecting member 16 through the first connecting member channel 1311 in the first fixing member body 131.

In other embodiments, part of the puncture needle 22 is received and fixed in the first fixing member 131, and part of the puncture needle 22 passing out of the first fixing member body 131 is communicated with the first connecting member 16. That is, the proximal end of the puncture needle 22 is directly communicated with the first connecting member 16. The first liquid medium can be directly input into the puncture needle 22 through the first connecting member 16, which improves the efficiency of inputting the first liquid medium into the puncture needle 22.

The first connecting member 16 includes a first connecting portion 162 and a first switch portion 161 connected to the first connecting portion 162. The first switch portion 161 is arranged outside the handle assembly 10. The proximal end of the first connecting portion 162 is connected with the first switch portion 161. The distal end of the first connecting member 162 is connected with the first connecting member channel 1311, and the first connecting member 162 is communicated with the first connecting member channel 1311.

For example, when the first switch portion 161 is in a normally open state, the first liquid medium or guide wire can be introduced through the first switch portion 161. The first liquid medium or guide wire is transferred to the first connecting member channel 1311 through the first connecting member 162, and then enters the body through the inner cavity of puncture needle 22. It can be understood that the first liquid medium is not limited to a contrast agent or normal saline. When the first switch portion 161 is in a normally closed state, it can prevent blood from passing through. In this embodiment, the first switch portion 161 is a three-way valve, and the first connecting portion 162 is of a hollow tubular structure.

The first fixing member body 131 is also provided with a second channel 1312 spaced apart from the first connecting member channel 1311. That is, the first connecting member channel 1311 and the second channel 1312 are not communicated. An inner diameter of the second channel 1312 is greater than that of the first connecting member channel 1311. The puncture assembly 20 also includes a second connecting member 17. The second connecting member 17 is also arranged outside the handle assembly 10. Part of the second connecting member 17 is connected with the outer tube body 21 through the second channel 1312.

Referring to Fig. 1, Fig. 3 and Fig. 5 again, in this embodiment, the outer tube body 21 can slide relative to the handle assembly 10 in the axial direction. That is, the distal end 21a of the outer tube body 21 can slide away from the handle assembly 10 in the axial direction, so that the puncture assembly 20 can be switched from the extending state to the receiving state. The distal end 21a of the outer tube body 21 can also slide close to the handle body 11, so that the puncture needle 22 can be switched from the receiving state to the extending state.

For example, referring to Fig. 2, the outer tube 23 also includes a second fixing member 15 located on a distal side of the first fixing member 13. The second fixing member 15 is used for fixing the outer tube body 21. The second fixing member 15 is received in the receiving member 113. The second fixing member 15 and the first fixing member 13 are spaced apart from each other. The second fixing member 15 is located on the distal side of the first fixing member 13, that is, in the axial direction, the proximal end of the first fixing member 13 is closer to the proximal end of the puncture device 1 than the second fixing member 15. The first fixing member 13 is fixedly connected with the proximal end 22b of the puncture needle 22, so that the distal end 22a of the puncture needle 22 can be controlled to extend out of the distal end 21a of the outer tube body 21 or into the distal end 21a of the outer tube body 21 through the first fixing member 13.

Referring to Fig. 1, Fig. 2 and Fig. 7, the handle assembly 10 also includes a second control element 14. The second control element 14 and the first control element 12 are spaced apart from each other. The second control element 14 is connected with the outer tube body 21. The second control element 14 is used for controlling the outer tube body 21 to slide axially relative to the puncture needle 22.

In this embodiment, the outer tube body 21 can slide relative to the handle body 11, so that it is convenient for accurately adjusting the position of the outer tube body 21 in the body. The second control element 14 controls the outer tube body 21 to slide axially relative to the handle body 11, so that the outer tube body slides away from the proximal end of the handle body 11 and covers the puncture needle 22 again. The puncture needle 22 is received in the hollow tube body of the outer tube body 21, thereby lowering the risk that the puncture needle 22 stabs the inner wall of the body and improving the safety of the operation.

In this embodiment, the first control element 12 is closer to the proximal end of the puncture device 1 than the second control element 14. After the first control element 12 controls the puncture needle 22 to be switched from the receiving state to the extending state and break through the atrial septum, it is necessary to control the second control element 14 to slide away from the proximal end of the puncture device 1, so as to control the outer tube body 21 to cover the puncture needle 22 again. Since the first control element 12 is closer to the proximal end of the puncture device 1 than the second control element 14, that is, since the second control element 14 is farther away from the proximal end of the puncture device 1 than the first control element 12, the second control element 14 is controlled to slide away from the proximal end of the puncture device 1, and the second control element 14 will not gradually move close to the first control element 12, but will gradually move far away from the first control element 12. Therefore, it is not easy to miscontact the first control element 12 in the process of controlling the second control element 14, avoiding that the position of the puncture needle 22 in the body is changed due to the miscontact with the first control element 12, and avoiding that the position of the puncture needle 22 is changed due to the miscontact with the first control element 12. If the first control element 12 is withdrawn after passive contact, the puncture needle 22 will retract into the outer tube body 21, so that the distal end of the puncture assembly 20 rebounds to the right atrium and cannot enter the left atrium.

In combination with Fig. 4a, Fig. 4b and Fig. 4c, the second control element 14 covers part of the first handle piece 111 and part of the second handle piece 112. The first handle piece 111 and the second handle piece 112 are both provided with notches 200. The notches 200 are formed by radial inwards recessing of part of the outer surface of the first handle piece 111 or part of the outer surface of the second handle piece 112. The second control element 14 is placed on the notch 200. Two opposite side walls of the notch 200 are used for restraining the axial travel of the second control element 14.

Referring to Fig. 2 and Fig. 7, the second control element 14 includes a second clamping portion 141 and a connecting thread 142 spaced apart from the second clamping portion 141. The second clamping portion 141 is a groove arranged on an inner surface of the second control element 14 and recessed towards the outer surface of the second control element 14. The second clamping portion 141 in the second control element 14 is used for cooperating with the second fixing member 15 to control the second fixing member 15 to axially move. The connecting thread 142 of the second control element 14 is used for adjusting the deformation and bending of the outer tube body 21. In this embodiment, both the second clamping portion 141 and the connecting thread 142 are arranged on the inner surface of the second control element 14, and the connecting thread 142 and the second clamping portion 141 can move axially with the second control element 14 at the same time. In other embodiments, the connecting thread 142 and the second clamping portion 141 can be respectively arranged in different sub-components in the second control element 14, as long as the connecting thread 142 and the second clamping portion 141 can move in the axial direction synchronously.

In this embodiment, the second control element 14 is connected with the proximal end of the outer tube body 21, and the second control element 14 is indirectly connected with the proximal end of the outer tube body 21. In other embodiments, the second control element 14 can also be directly connected with the proximal end of the outer tube body 21.

Referring to Fig. 4a, Fig. 4b and Fig. 4c, in this embodiment, the handle assembly 10 is provided with a second limiting hole 1113. The second limiting hole 1113 is used for restraining the axial travel of the second control element 14. For example, the first handle piece 111 in the handle assembly 10 is provided with a second limiting hole 1113. The second limiting hole 1113 and the first limiting hole 1111 are spaced apart from each other. The second limiting hole 1113 runs from the outer wall of the handle body 11 to the receiving member 113 in the handle body 11. Part of the second control element 14 is connected with the proximal end of the outer tube body 21 through the first limiting hole 1111. At least part of the proximal end of the outer tube body 21 directly faces the second limiting hole 1113. The second handle piece 112 is also provided with a second limiting hole 1113. The second limiting hole 1113 of the second handle piece 112 directly faces the second limiting hole 1113 of the first handle piece 111.

Referring to Fig. 2, the second fixing member 15 includes a second fixing member body 151. The proximal end of the outer tube body 21 extends into the second fixing member body 151 and is fixedly connected with the second fixing member body 151.

In this embodiment, the second fixing member 15 also includes a second fixing portion 152 connected with the second fixing member body 151. The second fixing portion 152 is connected with an outer wall of the second fixing member body 151. The second fixing portion 152 is connected with the second control element 14 through the second limiting hole 1113. For example, in combination with Fig. 4b, Fig. 4c and Fig. 7, the second fixing portion 152 protrudes radially from the outer wall of the second fixing member body 151. The second fixing portion 152 passes through the second limiting hole 1113 and is fastened with the second clamping portion 141 in the second control element 14 In the process of axial movement of the second control element 14, a distal side wall and proximal side wall of the second clamping portion 141 clamp a distal outer wall and proximal outer wall of the second fixing portion 152, and drive the second fixing portion 152 and the second fixing member body 151 to move axially. In this embodiment, part of the second fixing portion 152 directly faces the second limiting hole 1113.

Referring to Fig. 4a, Fig. 4b and Fig. 4c, in this embodiment, the second limiting hole 1113 includes a third limiting portion 1113a and a fourth limiting portion 1113b opposite to the third limiting portion 1113a. The third limiting portion 1113a and the fourth limiting portion 1113b are arranged at intervals in the axial direction. The third limiting portion 1113a and the fourth limiting portion 1113b restrain the axial travel of the second control element 14. The third limiting portion 1113a is a distal end of the second limiting hole 1114, and the fourth limiting portion 1113b is a proximal end of the first limiting hole 1113.

On the one hand, referring to Fig. 3 and Fig. 4b, when the second control element 14 slides from the proximal end of the second limiting hole 1113 to the distal end of the second limiting hole 1113, the second control element 14 drives the second fixing portion 152 and the outer tube body 21 to slide away from the proximal end 11b of the handle body 11. The outer tube body 21 gradually covers the distal part 22a of the puncture needle 22. When the second fixing portion 152 abuts against the distal end of the second limiting hole 1114, the second fixing portion 152 is restrained by the distal end of the second limiting hole 1114, and the second fixing portion 152 and the puncture needle 22 cannot continue to slide away from the proximal end 11b of the handle body 11. The second fixing portion 152 and the outer tube body 21 cannot continue to slide away from the proximal end 11b of the handle body 11, either. In this process, the puncture needle 22 is switched from the extending state to the receiving state.

On the other hand, referring to Fig. 4b and Fig. 5, when the second control element 14 slides from the distal end of the second limiting hole 1113 to the proximal end of the second limiting hole 1113, the second control element 14 drives the second fixing portion 152 and the outer tube body 21 to slide towards the proximal end 11b of the handle body 11, thus enabling the distal end 22a of the puncture needle 22 to be gradually exposed from the outer tube body 21. When the second fixing portion 152 abuts against the proximal end of the second limiting hole 1113, the second fixing portion 152 is restrained by the proximal end of the second limiting hole 1113, and the second fixing portion 152 and the puncture needle 22 cannot slide towards the proximal end of the handle body 10. In this process, the puncture needle 22 is switched from the receiving state to the extending state.

The third limiting portion 1113a and the fourth limiting portion 1113b are two opposite hole walls of the second limiting hole 1113 respectively. Other hole walls of the second limiting hole 1113 form the second limiting hole 1113 with the third limiting portion 1113a and the fourth limiting portion 1113b. A cross-sectional area of the second fixing member body 151 is larger than that of the second limiting hole 1113, so that the second fixing member body 151 cannot pass through the second limiting hole 1113, making the second fixing member body 151 not easy to leave the handle body 11.

In this embodiment, the axial travel of movement of the outer tube body 21 is greater than or equal to the axial travel of movement of the puncture needle 22. In this way, after the puncture needle 22 punctures the atrial septum, the outer tube body 21 slides away from the proximal end of the handle assembly 10, which can completely cover the puncture needle 22 again, thus lowering the risk that the puncture needle 22 stabs the inner wall of the body and improving the safety of the operation.

The axial travel of movement of the outer tube body 21 is an axial distance D2 between the third limiting portion 1113a and the fourth limiting portion 1113b. The axial travel of movement of the puncture needle 22 is an axial distance D1 between the first limiting portion 1111a and the second limiting portion 1111b. D2 is greater than or equal to D1, so that the axial travel of movement of the outer tube body 21 is greater than the axial travel of movement of the puncture needle 22, and the outer tube body 21 can cover the puncture needle 22 again after the puncture needle 22 extends out of the outer tube body 21. The axial travel of movement of the outer tube body 21 is the same as the axial travel of movement of the second control element 14. The axial travel of movement of the puncture needle 22 is the same as the axial travel of movement of the first control element 12.

In this embodiment, when the outer tube body 21 slides away from the proximal end 11b of the handle body 11, the puncture needle 22 remains stationary relative to the proximal end 11b of the handle body 11, so as to ensure that the position of the puncture needle 22 in the body remains unchanged, and prevent the puncture needle 22 from moving and easily stabbing the left atrial wall.

Referring to Fig. 2, Fig. 6a, Fig. 8a and Fig. 8b, the second fixing member 15 is provided with a communicating cavity 1512 communicated with the inner cavity of the outer tube body 21, and a puncture member channel 1511 communicated with the communicating cavity 1512. The puncture needle 22 is threaded in the outer tube body 21 through the puncture member channel 1511 and the communicating cavity 1512. For example, the second fixing member body 151 includes a puncture member channel 1511. The proximal end of the outer tube body 21 is inserted into the second fixing member body 151. The puncture member channel 1511 is opposite to the first connecting member channel 1311. The puncture needle 22 passes through the puncture member channel 1511 and is inserted into the outer tube body 21.

In this embodiment, the second fixing member body 151 also includes a communicating cavity 1512 and a second connecting member channel 1513. The second connecting member channel 1513 is spaced apart from the puncture member channel 1511, and the second connecting member channel 1513 is communicated with the communicating cavity 1512. The communicating cavity 1512 is communicated with the puncture member channel 1511. The second connecting member channel 1513 is communicated with the puncture member channel 1511 through the communicating cavity 1512.

Referring to Fig. 1, Fig. 2 and Fig. 6a, the second connecting member 17 can be used for introducing a second liquid medium. The second liquid medium can be delivered into the body through the second connecting member 17, the second connecting member channel 1513, the communicating cavity 1512 and the outer tube body 21 in sequence. For example, the second connecting member 17 includes a second switch portion 171 and a second connecting portion 172 connected to the second switch portion 171. The second switch portion 171 is arranged outside the handle body 11. The proximal end of the second connecting member 17 is connected to the second switch portion 171. Part of the second connecting portion 172 passes through the second channel 1312. The distal end of the second connecting portion 172 is connected with the second connecting member channel 1513 and the communicating cavity 1512. When the second switch portion 171 is in a normally open state, the second liquid medium can be introduced through the second switch portion 171. The second liquid medium is transferred to the second connecting member channel 1513, the communicating cavity 1512 and the inner cavity of the outer tube body 21 through the second connecting portion 172 and enters the body. It can be understood that the second liquid medium is not limited to physiological saline and the like, which can be determined according to actual needs.

On the one hand, in the process of discharging air from the puncture device 1, the first liquid medium flows through the first connecting member 16, the first connecting member channel 1311 and the puncture needle 22 to discharge the air in the first connecting member 16, the first connecting member channel 1311 and the puncture needle 22 out of the puncture device 1. The second liquid medium flows through the second connecting member 17, the second connecting member channel 1513, the communicating cavity 1512 and the outer tube body 21, and discharges the air in the second connecting member 17, the second connecting member channel 1513, the communicating cavity 1512 and the outer tube body 21 out of the puncture device 1. This embodiment can effectively discharge the air in the puncture device 1 out of the puncture device 1, so as to avoid air embolism during the operation. It can be understood that during the discharging of the air, the air in the puncture device 1 cannot be completely discharged if only the first liquid medium flows through the first connecting member 16, the first connecting member channel 1311 and the puncture needle 22, or only the second liquid medium flows through the second connecting member 17, the communicating cavity 1512 and the outer tube body 21. This is because the air discharging for the first connecting member 16, the first connecting member channel 1311 and the puncture needle 22 is independent of the air discharging for the second connecting member 17, the second connecting member channel 1513, the communicating cavity 1512 and the outer tube body 21.

On the other hand, when the first liquid medium is a contrast medium, the first liquid medium flows out of the distal end 22a of the puncture needle 22, which can accurately determine the position of the distal end 22a of the puncture needle 22, so that it is convenient for the doctor to control the puncture needle 22 to puncture the atrial septum, thus improving the accuracy of the puncture surgery.

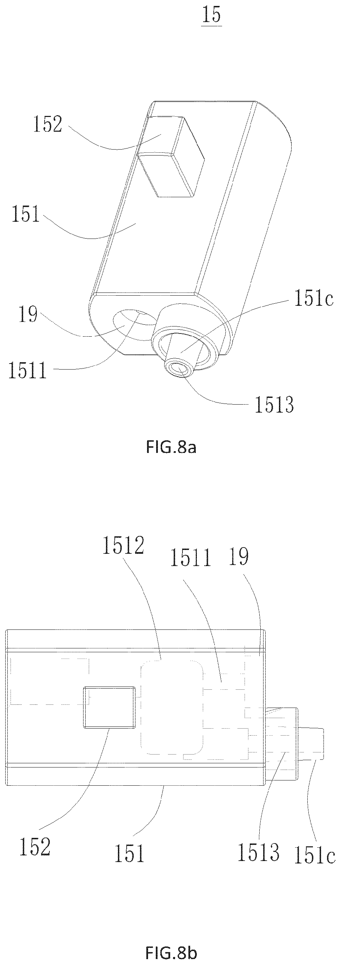

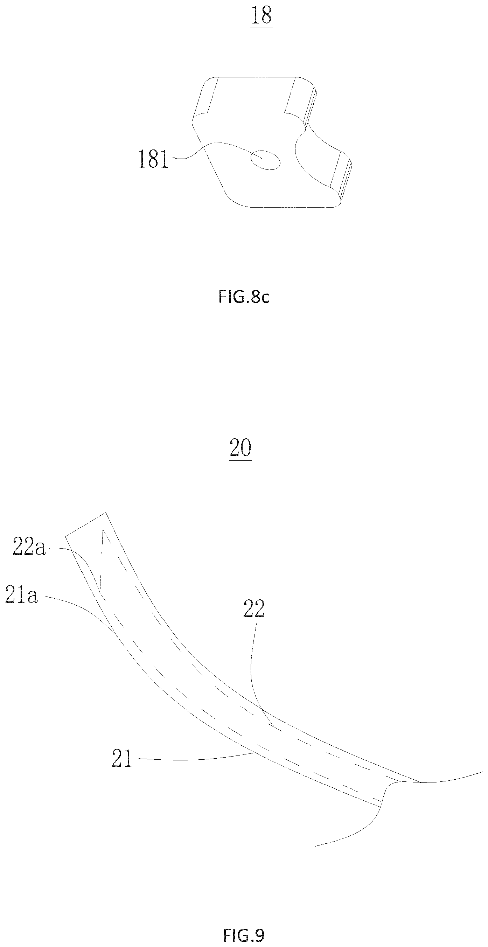

Referring to Fig. 6a, Fig. 8a, Fig. 8b and Fig. 8c, the proximal end of the second fixing member body 151 is provided with a communicating slot 19. The communicating slot 19 is communicated with the puncture member channel 1511. The proximal end of the second fixing member body 151 is also provided with a connector 151c. The connector 151c is spaced apart from the puncture member channel 1511. The connector 151c is used for being connected with the second connecting portion 172. The second connecting portion 172 is threaded in the connector 151c and communicated with the second connecting member channel 1513.

Referring to Fig. 6a, Fig. 8a and Fig. 8c, the handle assembly 10 also includes a sealing member 18. The sealing member 18 is fitted and fixed to an end face of the proximal end of the second fixing member body 151, and seals an opening of the communicating slot 19 in the second fixing member body 151, so as to prevent the liquid from flowing through a gap between the communicating slot 19 and the sealing member 18. The way of fitting and fixing the sealing member 18 to the end face of the proximal end of the second fixing member body 151 is not limited to one or a combination of more of bonding, insertion and the like.

In this embodiment, the sealing member 18 is cubic as a whole. The sealing member 18 is provided with an opening 181. The opening 181 runs through an end face of the proximal end and an end face of the distal end of the sealing member 18, and the opening 181 is communicated with the communicating slot 19. The puncture needle 22 passes through the opening 181 and is connected with the puncture member channel 1511. An inner wall of the opening 181 is in interference fit with the puncture needle 22 to prevent the liquid from flowing through a space between the puncture needle 22 and the opening 181.

A side wall of part of the sealing member 18 is recessed towards the axial direction where the opening 181 is located. This portion is fitted to a side wall of the connector 151c in the second fixing member body 151, so that the sealing member 18 and the second fixing member body 151 are more compact.

Referring to Fig. 9, the puncture assembly 20 is bendable and deformable. For example, the outer tube body 21 in the puncture assembly 20 is bendable and deformable. Referring to Fig. 2 and Fig. 3, the puncture device 1 also includes a bending adjustment assembly 30. The bending adjustment assembly 30 is connected with the puncture assembly 20, and the bending adjustment assembly 30 is also connected with the handle assembly 10. The bending adjustment assembly 30 controls the bending deformation of the puncture assembly 20. In this embodiment, the outer tube body 21 adjusts its form through the bending adjustment assembly 30. In other embodiments, the outer tube body 21 can be prefabricated, that is, it is already bent before extending into the body.

For example, the bending adjustment assembly 30 includes an adjustment member 31, a bending adjustment member 33, and a traction member 32 connected between the adjustment member 31 and the bending adjustment member 33. The adjustment member 31 is received in the receiving member 113. The adjustment member 31 is spaced apart from the second fixing member 15, and is arranged on one side of the second fixing member 15 away from the first fixing member 13. The adjustment member 31 sleeves the outer wall of the outer tube body 21, and the adjustment member 31 can slide on the outer wall of the outer tube body 21. The bending adjustment member 33 is arranged in the distal end of the outer tube body 21 and fixed in the distal end of the outer tube body 21. A radial inward projection of the bending adjustment member 33 falls onto the distal end of the puncture needle 22. The proximal end of the traction member 32 is connected with the adjustment member 31. The distal end of the traction member 32 is connected with the bending adjustment member 33.

The adjustment member 31 is connected with the second control element 14. Referring to Fig. 2, Fig. 4a, Fig. 4b and Fig. 4c, at least one of the first handle piece 111 and the second handle piece 112 is provided with a third limiting hole 300. The third limiting hole 300 and the second limiting hole 1113 are spaced apart from each other. The third limiting hole 300 runs from the outer wall of the handle body 11 to the receiving member 113 in the handle body 11. Part of the adjustment member 31 extends out of the third limiting hole 300 and are screwed to the connecting thread 142 in the second control element 14.

The specific process that the second control element 14 adjusts the position of the adjustment member 31 to bend the outer tube body 21 is described as follows. For example, the second control element 14 abuts against a side wall of the proximal end of the notch 200, takes the side wall of the proximal end of the notch 200 as a support, and rotates relative to the axis of the handle body 11, and the adjustment member 31 slides on the outer wall of the outer tube body 21 relative to the handle body 11 as the second control element 14 rotates. When the second control element 14 drives the adjustment member 31 to slide towards the proximal end of the outer tube body 21, the adjustment member 31 pulls the traction member 32 and the bending adjustment member 33 to move close to the proximal end of the outer tube body 21, so that the distal end of the outer tube body 21 is bent towards the proximal end of the outer tube body 21. When the outer tube body 21 is bent towards the proximal end of the outer tube body 21, the puncture needle 22 will also be bent in the same direction. In this embodiment, the bending adjustment member 33 is of a hollow ring structure, and the bending adjustment member 33 can be used as a developing point.

In this embodiment, the outer tube body 21 is bendable and deformable to facilitate the adjustment of the form of the outer tube body 21 in the body, so as to make the distal end of the outer tube body 21 face a predetermined position, so that the puncture needle 22 can accurately puncture the predetermined position, which improves the puncture accuracy of the puncture needle 22.

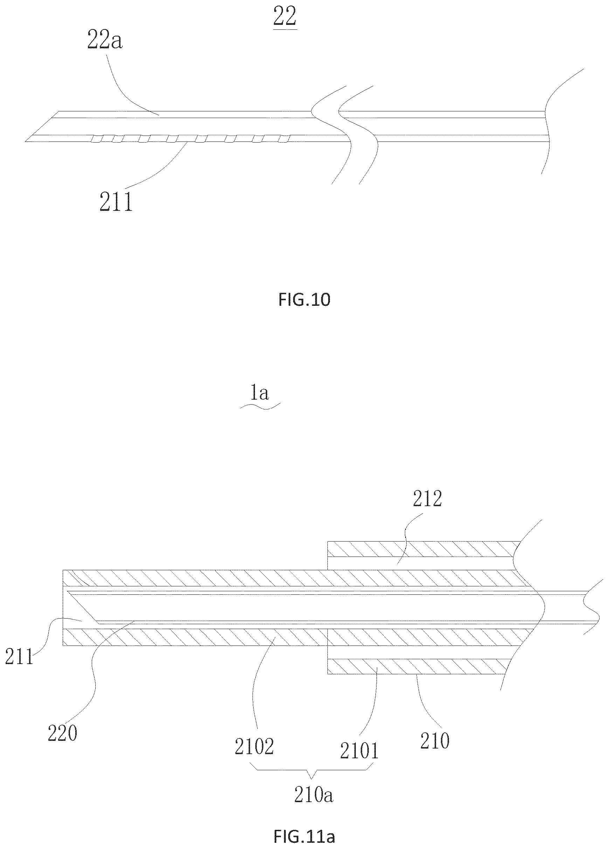

In this embodiment, the puncture needle 22 is bendable and deformable. For example, the distal end 22a of the puncture needle 22 is provided with a bendable structure 211. Referring to Fig. 10, the bendable structure 211 can bend and deform the distal end 22a of the puncture needle 22, so as to adjust the orientation of the end face of the distal end of the puncture needle 22. The bendable structure 211 directly faces the distal end of the outer tube body 21. When the puncture assembly 20 is in the receiving state, the bendable structure 211 is received in the distal end of the outer tube body 21. It can be understood that the bendable structure 211 may be helical or dovetail-shaped. It can be understood that in other embodiments, the shape of the bendable structure 211 is not limited as long as the distal end of the puncture needle 22 is bendable. In this embodiment, the form of the puncture needle 22 is adjusted through the bending adjustment assembly 30. In other embodiments, the puncture needle 22 can be prefabricated to be deformed, that is, it has been already bent before extending into the body.

In this embodiment, the puncture needle 22 is a hollow tube. The end face of the distal end 22a of the puncture needle 22 is recessed towards the proximal end of the puncture needle 22 to form an inclined puncture surface. The puncture surface is provided with a sharp corner to facilitate stress concentration and improve the puncture efficiency.

### Second embodiment

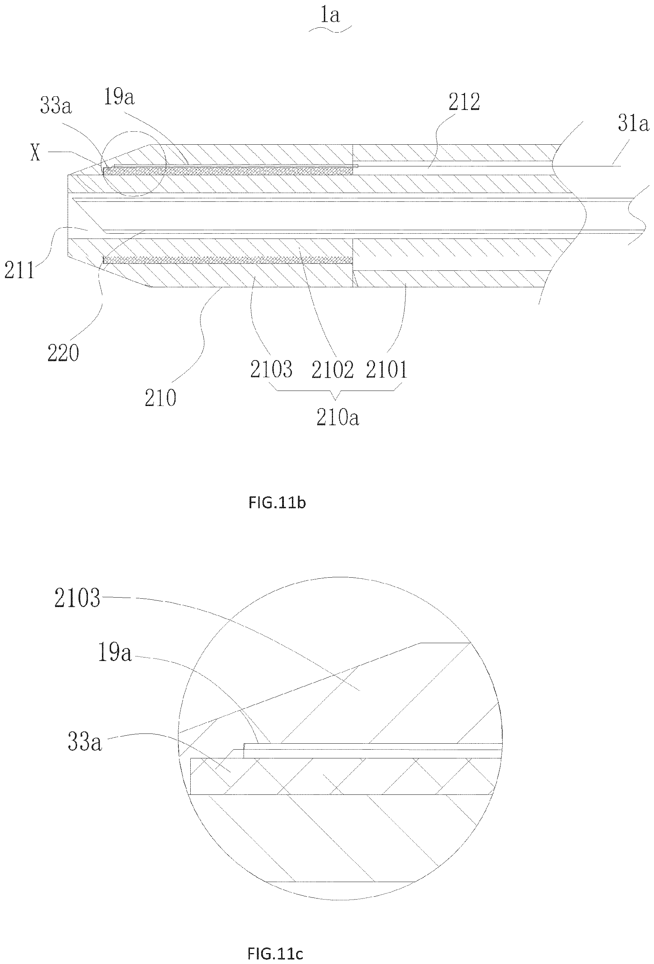

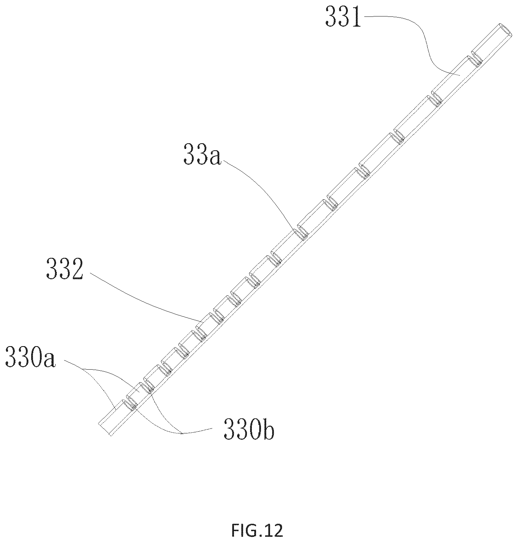

Referring to Fig. 11a, Fig. 11b, Fig. 11c and Fig. 12, the puncture device 1a provided in this embodiment is basically the same as that in the first embodiment, but a difference is that in this embodiment, the bending adjustment member 33a is elastic. The bending adjustment member 33a is a hollow tubular knot. The bending adjustment member 33a is arranged in the distal end 210a of the outer tube body 210. The traction member 31a is connected between an adjustment member (not shown in the figure) and the bending adjustment member 33a.

The handle assembly includes a bending adjustment control element (not shown in the figure), and the bending adjustment control element (i.e. the second control element in the first embodiment and a handling member in the fifth embodiment) is used for controlling the distal end of the outer tube body 21 to be bent. The specific structure of the bending adjustment control element can refer to the second control element in the first embodiment. The bending adjustment control element is screwed with the adjustment member, and the bending adjustment control element rotates relative to the handle assembly (not shown in the figure), which can drive the adjustment member and the traction member 31a to slide relative to the handle assembly. For example, when the bending adjustment control element rotates in a first rotating direction relative to the axial central axis of the handle assembly, the bending adjustment control element drives the adjustment member and the traction member 31a to slide towards the proximal end of the handle assembly, and the bending adjustment member 33a is bent towards the proximal end of the handle assembly in a first bending direction, so that the distal end of the outer tube body 210 and the distal end of the puncture needle 220 are bent in the first bending direction towards the proximal end of the handle assembly. When the bending adjustment control element rotates in a second rotating direction (opposite to the first rotating direction) relative to the axial central axis of the handle assembly, the bending adjustment control element drives the adjustment member and the traction member 31a to slide away from the proximal end of the handle assembly, and the bending adjustment member 33a gradually recovers to its original shape, so that the distal end of the outer tube body 210 and the distal end of the puncture needle 220 are flush or nearly flush with the axial central axis of the handle assembly. It can be understood that the traction member 31a is made of an elastic metal or non-metal material such as a nickel titanium wire.

In this embodiment, the distal end 210a of the outer tube body 210 includes a main body portion 2101 and a supporting portion 2102 connected with the main body portion 2101. The supporting portion 2102 protrudes along the proximal end of the outer tube body 210. The bending adjustment member 33a sleeves an outer surface of the supporting portion 2102. A radially inwards projection of the bending adjustment member 33a falls onto a radially inwards projection of the supporting portion 2102.

The outer tube body 210 is provided with a first through hole 211. The first through hole 211 runs through the proximal end of the outer tube body 210 and the distal end 210a of the outer tube body 210. That is, the first through hole 211 runs through the main body portion 2101 and the supporting portion 2102. The first through hole 211 is used for receiving the puncture needle 220.

The outer tube body 210 is also provided with an outer tube channel 212. The outer tube channel 212 is spaced apart from the first through hole 211. The outer tube channel 212 runs through the main body portion 2101. The outer tube channel 212 is arranged in the outer tube body 210, and an extending direction of the outer tube channel 212 is the same as that of the first through hole 211. The distal end of the traction member 31a extends to the bending adjustment member 33a along the outer tube channel 212 through a side wall of the outer tube body 210, and is fixedly connected with the bending adjustment member 33a. In other embodiments, the bend adjustment member 33a can be omitted. At this time, the traction member 31a is directly fixed at the distal end 210a of the outer tube body 210, and the traction member 31a pulls the distal end 210a of the outer tube body 210 to be bent. In other embodiments, the traction member 31a is directly fixed at the distal end 210a of the outer tube body 210 and is not connected with the bending adjustment member 33a. The traction member 31a pulls the distal end 210a of the outer tube body 210 to be bent under the action of an external force, and the bending adjustment member 33a is bent with the outer tube body 210. After the external force is removed, the bending adjustment member 33a restores to its original shape, and an elastic force for causing the bending adjustment member 33a to restore to its original shape also makes the outer tube body 210 restore to its original shape.

In this embodiment, the handle assembly also includes a spacer 19a. The spacer 19a is of a hollow tubular structure. The spacer 19a is arranged on an outer wall of the bending adjustment member 33a. The spacer 19a is made of a macromolecular material. Part of the traction member 31a is received in the spacer 19a. The distal end of the traction member 31a passes through an inner cavity of the spacer 19a and is fixedly connected with the supporting portion 2102.

In this embodiment, the distal end 210a of the outer tube body 210 also includes a covering portion 2103. The covering portion 2103 covers the spacer 19a, and the covering portion 2103 covers the portion of the supporting portion 2102 exposed from the main body portion 2101. The proximal end of the covering portion 2103 is flush with the distal end of the main body portion 2101, so as to avoid a height difference formed between the covering portion 2103 and the main body portion 2101, which may affect the withdrawal of the outer tube body 210 from the body.

The covering portion 2103 also covers an outer wall of the bending adjustment member 33a, so that the bending adjustment member 33a is firmly received in the outer tube body 210, which prevents the bending adjustment member 33a from falling off from the outer tube body 210.

In this embodiment, the spacer 19a separates the traction member 31a from the covering portion 2103, and the portion of the traction member 31a in the inner cavity of the spacer 19a can move relative to the spacer 19a, thus preventing such a phenomenon that the supporting portion 2102 cannot be bent since the portion of the traction member 31a covered by the covering portion 2103 cannot slide relative to the spacer 19a due to abutment and limitation of the covering portion 2103.

In this embodiment, a material of the covering portion 2103 is the same as the material of the main body portion 2101, both of which are macromolecular materials. The molten covering portion 2103 may be fused with the main body portion 2101. In other embodiments, a material of the covering portion 2103 may be different from the material of the main body portion 2101.

In this embodiment, a melting point of the covering portion 2103 is lower than that of the spacer 19a. In the process of fusing the molten covering portion 2103 with the main body portion 2102, the spacer 19a will not be melted, thus preventing the fused covering portion 2103 from fully fixedly connecting a traction wire 31a in the spacer 19a to the spacer 19a into a whole. In this embodiment, the proximal end of the spacer 19a is farther away from the distal end of the outer tube body 210 than the proximal end of the bending adjustment member 33a, which prevents the covering portion 2103 from covering an end face of the proximal end of the spacer 19a, and more effectively prevents the fused covering portion 2103 from fully fixedly connecting the traction wire 31a in the spacer 19a to the spacer 19a into a whole.

In this embodiment, the distal end of the traction member 31a passes through the distal end of the spacer 19a, and is covered and fixed by the covering portion 2013. When the traction member 31a is pulled, the traction member 31a can slide towards the proximal end of the handle assembly in the spacer 19a, and the distal end of the traction member 31a pulls the covering portion 2103, so that the bending adjustment member 33a and the supporting portion 2102 are bent towards the proximal end of the handle assembly. In other embodiments, the distal end of the traction member 31a passes through the spacer 19a and is then fixedly connected with the bending adjustment member 33a. When the traction member 31a is pulled, the distal end of the traction member 31a pulls the bending adjustment member 33a, so that the bending adjustment member 33a and the supporting portion 2102 are bent towards the proximal end of the handle assembly.

Referring to Fig. 12, the outer wall of the bending adjustment member 33a is provided with a plurality of elastic portions 330a and a plurality of opening portions 330b. Each opening portion 330b is connected between two adjacent elastic portions 330a. Each opening portion 330b is of a groove structure formed in the bending adjustment member 33a. The opening portion 330b can reduce the rigidity of the bending adjustment member 33a, so that the bending adjustment member 33a is bent more easily.

Further, when the second control element drives the adjustment member 31 to slide towards the proximal end of the outer tube body 210, adjacent elastic portions 330a move close to each other, and the opening portion 330b between the adjacent two elastic portions 330a is gradually narrowed, so that the entire bending adjustment member 33a is deformed and bent.

In this embodiment, the distribution number of opening portions 330b per unit length increases gradually from the proximal end 331 of the bending adjustment member to the distal end 332 of the bending adjustment member. The rigidity of the bending adjustment member 33a decreases gradually from the proximal end 331 of the bending adjustment member to the distal end 332 of the bending adjustment member. The bending adjustment member 33a is bent more easily from the proximal end 331 of the bending adjustment member to the distal end 332 of the bending adjustment member. In other embodiments, the distribution number of opening portions 330b per unit length remains unchanged from the proximal end 331 of the bending adjustment member to the distal end 332 of the bending adjustment member.

### Third embodiment

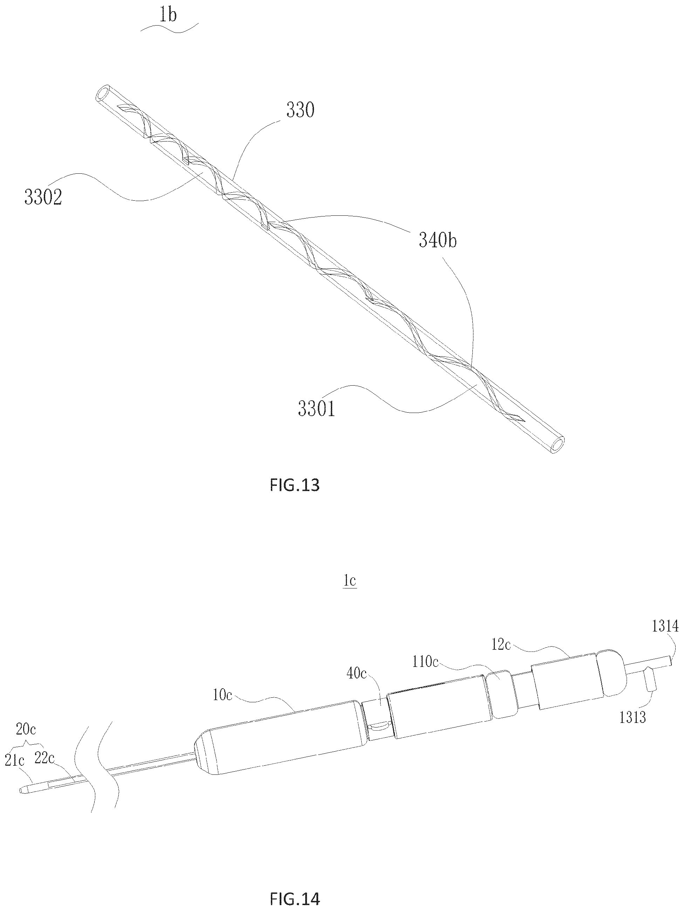

Referring to Fig. 13, the puncture device 1b provided in this embodiment is basically the same as that in the second embodiment. A difference is that a plurality of opening portions 340b are connected and spirally extend along the outer wall of the bending adjustment member 330 to form a whole.

A pitch of two adjacent opening portions 340b gradually decreases from the proximal end 3301 of the bending adjustment member to the distal end 3302 of the bending adjustment member. The rigidity of the bending adjustment member 330 decreases gradually from the proximal end 3301 of the bending adjustment member to the distal end 3302 of the bending adjustment member, and the bending adjustment member 330 is bent more easily from its proximal end 3301 to its distal end 3302. In other embodiments, a pitch of the opening portions 340b remains unchanged from the proximal end 3301 of the bending adjustment member 330 to the distal end 3302.

### Fourth embodiment

The puncture device 1c provided in this embodiment is basically the same as that in the second embodiment, in which, components such as the bending adjustment assembly, the second control element and the adjustment assembly are basically the same as those in the second embodiment. The process of adjusting the bending of the distal end of the puncture member is basically the same as that in the second embodiment, and will not be repeated here. In this embodiment, a difference from the second embodiment is that the first control element, the first fixing member, the second fixing member and other components are described in detail below.

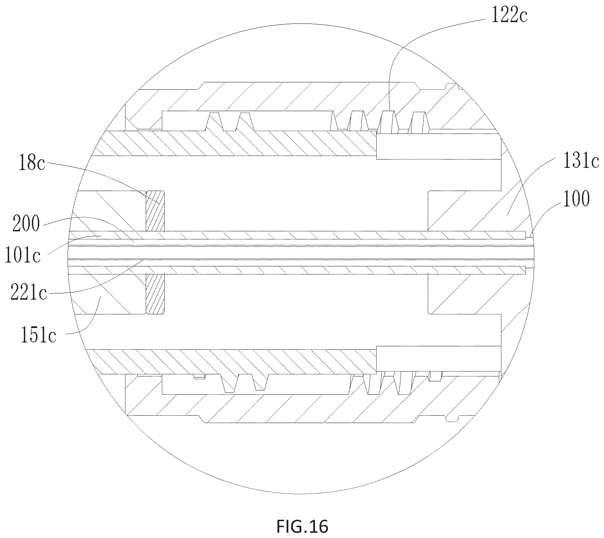

Referring to Fig. 14 to Fig. 19, the puncture device 1c includes a handle assembly 10c, and a puncture assembly 20c received in the handle assembly 10c and fixedly connected to the handle assembly 10c. The puncture assembly 20c includes a puncture needle 22c. The handle assembly 10c also includes a first control element 12c for controlling the puncture needle 22c. When the first control element 12c rotates, the first control element 12c controls the puncture needle 22c to move close to or away from the handle assembly 10c. The first control element 12c controls the puncture needle 22c to move close to or away from the handle assembly 10c, so that the first control element 12c can control the puncture needle 22c to extend out of the handle assembly 10c or extend into the handle assembly 10c, which is easy to operate and convenient for controlling the puncture needle 22c to perform the puncture surgery. The puncture needle 22c can be prevented from continuously moving and harming the body due to inertia after a successful puncture during the traditional puncture surgery.

Referring to Fig. 14 to Fig. 17, the first control element 12c is connected with the proximal end portion 221c of the puncture needle 22c. Furthermore, the proximal end portion 221c of the puncture needle 22c is received in the first control element 12c. When the first control element 12c rotates, the proximal end portion 221c received in the first control element 12c is controlled to move close to or away from the handle assembly 10c, so that the puncture member 12c moves close to or away from the handle assembly 10c.

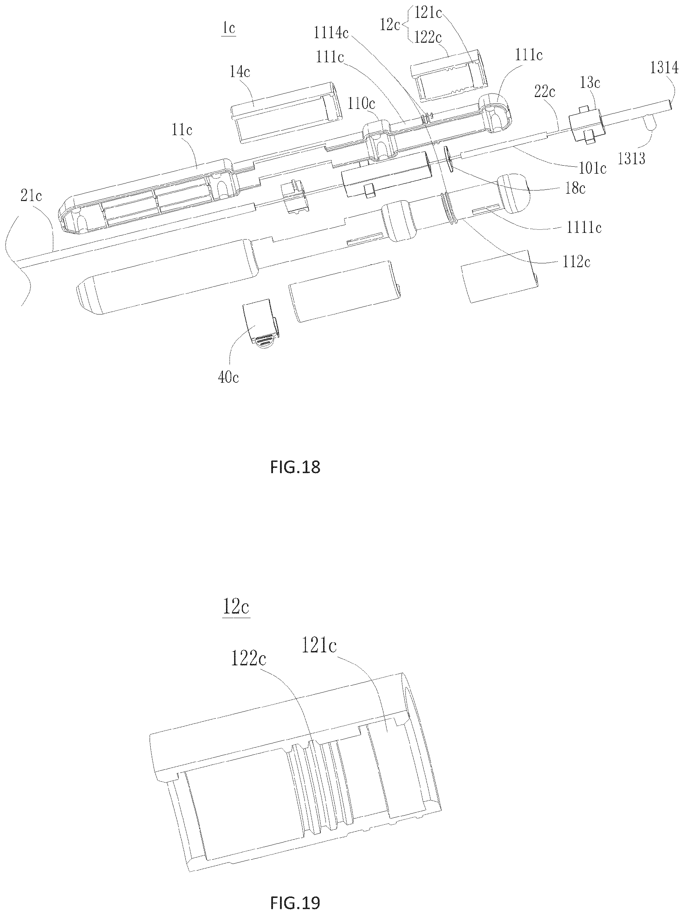

The first control element 12c and the handle body 11c can be in threaded connection. The first control element 12c sleeves the handle body 11c. The proximal end portion 221c of the puncture needle 22c is received in the handle body 11c, and the proximal end portion 221c is connected with the first control element 12c. A first thread portion 122c is formed on an inner surface of the first control element 12c. In Fig. 19, the inner surface of the first control element 12c is also provided with a first clamping portion 121c. The first thread portion 122c is arranged at a distal side of the first clamping portion 121c. The outer surface of the handle body 11c is provided with a second thread portion 1114c. The second thread portion 1114c is arranged at a distal side of the first limiting hole 1111c. In this embodiment, the handle body 11c includes a first handle piece 111c. The second thread portion 1114c is arranged on the outer surface of the first handle piece 111c and can be in threaded connection with the first thread portion 122c.

In this embodiment, the steps of operating the first control element 12c and the puncture needle 22c are as follows. First, the first control element 12c is subjected to a drive force parallel to the axial direction and facing away from the proximal end of the handle body 11c, so that the first control element 12c drives the first fixing member 13c and the puncture needle 22c connected to the first fixing member 13c to slide away from the proximal end of the handle body 11c, and the puncture needle 22c extends out of the outer tube body 21c. After the first thread portion 122c of the first control element 12c abuts against the second thread portion 1114c, the driving force parallel to axial direction and facing the distal end of the handle body 11c cannot make the first control element 12c to continue to slide. Then, a rotating force is applied to the first control element 12c to make the first control element 12c rotate circumferentially around the handle body 11c. The first thread portion 122c in the first control element 12c cooperates with the second thread portion 1114c. When the first control element 12c rotates axially around the handle body 11c, the first control element 12c continues to slide towards the distal end of the handle body 11c, and drives the first fixing member 13c and the puncture needle 22c connected to the first fixing member 13c to slide steadily away from the proximal end of the handle body 11c, and the puncture needle 22c punctures the fossa ovalis.

In combination with Fig. 14 to Fig. 17, during the puncture surgery, the puncture needle 22c can quickly extend out of the outer tube body 21c by pushing the first control element 12c. Then, by rotating the first control element 12c, a torque of rotating the first control element 12c is converted into a puncture acting force of the puncture needle 22c, which is convenient for the puncture needle 22c to puncture the fossa ovalis. The puncture process of the puncture needle 22c is more stable, so that it is not easy for the puncture needle to move and deviate from a target position, thus improving the accuracy of puncture by the puncture needle 22c. After the successful puncture, the puncture needle 22c does not easily continue to puncture under the inertia effect, so as to prevent the puncture needle 22c from stabbing the left atrial wall, and improve the safety of the puncture surgery by the puncture needle 22c.

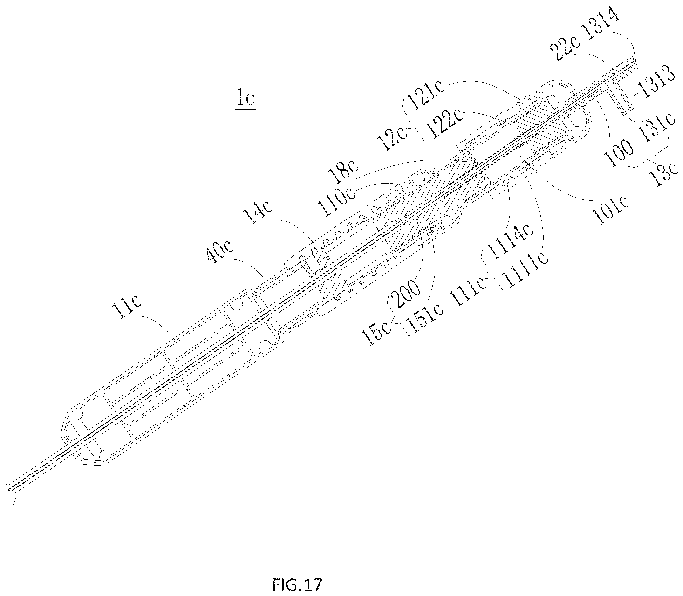

Referring to Fig. 17 and Fig. 18, the handle body 11c is provided with a first limiting hole 1111c. The first limiting hole 1111c is used for restraining an axial travel of the first control element 12c. An axial distance between the first thread portion 122c and the second thread portion 1114c is less than an axial length of the first limiting hole 1111c, so as to ensure that after the first control element 12c controls and drives the puncture needle 22c to slide a preset distance axially away from the proximal end of the handle body 11c, the first thread portion 111c can contact the second thread portion 1114c to connect the first thread portion 122c with the second thread portion 1114c.

The first handle piece 111c in this embodiment is basically the same as that in the second embodiment, so it will not be repeated here. A difference is that in this embodiment, the first handle piece 111c is provided with a limiting portion 110c. The limiting portion 110c protrudes from a surface of the first handle piece 111c away from the axial direction. The second control element 14c moves towards the proximal end of the handle body 11c in the axial direction. After the second control element moves the preset distance, the proximal end of the second control element 14c will abut against the limiting portion 110c and not continue to move.

In this embodiment, the first handle piece 111c includes two first sub-handle pieces (not shown in the figure), which are bolted to facilitate assembling and disassembling of the first handle piece 111c.

In other embodiments, the first thread portion 122c may be arranged on the proximal side of the first clamping portion 121c. After the first control element 12c rotates circumferentially around the handle body 11c to drive the first fixing member 13c and the puncture needle 22c connected to the first fixing member 13c to axially move, the first control element 12c continues to move close to or away from the handle assembly 10c in the axial direction, and drives the puncture needle 22c to move close to or away from the handle assembly 10c.

In other embodiments, the inner surface of the first control element 12c can be provided with only the first thread portion 122c, and the first thread portion 122c is in threaded connection with the outer wall of the handle body 11c. The first control element 12c rotates circumferentially around the handle body 11c to drive the first fixing member 13c and the puncture needle 22c connected to the first fixing member 13c to axially move.

In other embodiments, the inner surface of the first control element 12c can be provided with only the first clamping portion 121c, and the first clamping portion 121c cooperates with the first fixing member 13c. The first control element 12c moves close to or away from the handle assembly 10c in the axial direction, and drives the first clamping portion 121c, the first fixing member 13c, and the puncture needle 22c connected to the first fixing member 13c to move close to or away from the handle assembly 10c.

In other embodiments, the puncture needle 22c can be integrally molded with the first fixing member 13c, and the first control element 12c is connected with the puncture needle 22c and the first fixing member 13c. Part of the puncture needle 22c runs through the handle body 11c and is clamped to the first clamping portion 121c. The first control element 12c can drive the puncture needle 22c to move axially.

In other embodiments, the puncture assembly 20c may include only the puncture needle 22c. The puncture device 1c is delivered to a designated position of the body through a delivery sheath. The first control 12c controls the movement of the puncture needle 22c relative to the handle assembly 11c, and moves out of the delivery sheath or into the delivery sheath of the delivery device.

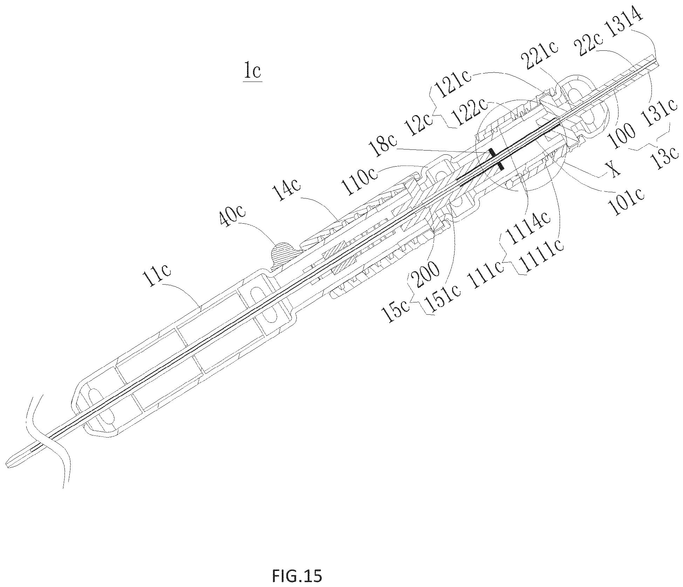

Referring to Fig. 15, Fig. 16 and Fig. 17, the first fixing member body 131c in the first fixing member 13c is provided with a first channel 100. The first channel 100 runs through the distal end of the first fixing member body 131c and the proximal end of the first fixing member body 131c in the axial direction.

The second fixing member body 151c in the second fixing member 15c is provided with a second channel 200. The second channel 200 runs through the distal end of the second fixing member body 151c and the proximal end of the second fixing member body 151c in the axial direction. In this embodiment, the first channel 100 and the second channel 200 are opposite. The proximal end of the outer tube body 21c is fixed to the distal end of the second fixing member body 151c, and the proximal end of the outer tube body 21c extends into the second channel 200 and is communicated with the second channel 200.

In this embodiment, the puncture assembly 20c also includes a sealing member 18c. The sealing member 18c seals the proximal end of the second fixing member body 151c. That is, the sealing member 18c seals the proximal side of the second channel 200, to prevent air or liquid from flowing out of the second fixing member body 151c from an opening at the proximal end of the second fixing member body 151c.

The puncture assembly 20c also includes a connecting member 101c. The connecting member 101c is of a hollow tubular structure. The proximal end of the connecting member 101c extends into the first channel 100 and is fixed at the distal end of the first fixing member body 131c. The proximal end of the connecting member 101c can be fixed at the distal end of the first fixing member body 131c by bonding. A bonding material fills a space between the outer wall of the connecting member 101c and the inner wall of the first channel 100, which prevents air or liquid from flowing out of the first fixing member body 131c from the space between the outer wall of the connecting member 101c and the inner wall of the first channel 100.

The distal end of the connecting member 101c extends into the second channel 200 and is communicated with the second channel 200 after passing through the sealing member 18c. The connecting member 101c communicates the first channel 100 with the second channel 200. The connecting member 101c is slidably connected in the second channel 200. The distal end of the connecting member 101c may slide with the sliding of the first fixing member 13c. In the process of sliding in the second channel 200, the connecting member 101c will not be separated from the second channel 200, ensuring that the first channel 100, the connecting member 101c and the second channel 200 remain connected.

The puncture needle 22c passes through the first channel 100 and the second channel 200. The proximal end of the puncture needle 22c extends into the first channel 100 and is fixed in the proximal end of the first fixing member body 131c. The puncture needle 22c passes through the inner cavity of the connecting member 101c, and extends along the second channel 200 into the inner cavity of the outer tube body 21c.

Referring to Fig. 15 to Fig. 17, in this embodiment, the proximal end of the first fixing member body 131c includes a first inlet 1313 and a second inlet 1314 spaced apart from the first inlet 1313. The second inlet 1314 is communicated with the inner cavity of the puncture needle 22c. The first inlet 1313 is communicated with the first channel 100.

In the process of discharging air of the puncture device 1c, a first liquid medium flows in the outer tube body 21c from the first inlet 1313 through the first channel 100, the connecting member 101c and the second channel 200, and is then discharged out of the puncture device 1c from the distal end of the outer tube body 21c. This part of first liquid medium brings away the air in the outer tube body 21c.

A second liquid medium flows in from the second inlet 1314. The second liquid medium flows through the puncture needle 22c and is discharged out of the puncture device 1c through the distal end of the puncture needle 22c to discharge the air in the puncture needle 22c. This embodiment can effectively discharge the air of the puncture needle 22c, the air of the outer tube body 21c, the air of the first fixing member 13c and the air of the second fixing member 15c in the puncture device 1c out of puncture device 1c to avoid air embolism during the operation. During the contrasting, a contrast agent flows in from the second inlet 1314 and is discharged out of the puncture device 1c through the distal end of the puncture needle 22. The contrast agent is discharged from the distal end of the puncture needle 22, which is convenient for obtaining the position of the distal end of the puncture needle 22, so that it is convenient for a doctor to conduct the puncture surgery, and the accuracy of the surgery is improved.

In other embodiments, the proximal end of the first fixing member body 131c may include only the first inlet 1313. The first inlet 1313 is communicated with the inner cavity of the puncture needle 22c, and the first inlet 1313 is communicated with the first channel 100. The first liquid medium flows in from the first inlet 1313. Part of the first liquid medium enters the inner cavity of the puncture needle 22c. This part of the first liquid medium flows through the puncture needle 22c, and is discharged out of the puncture device 1c through the distal end of the puncture needle 22c, so as to discharge the air in the puncture needle 22c. The other part of the first liquid medium enters the first channel 100. This part of the first liquid medium flows through the connecting member 101c, the second channel 200 and the outer tube body 21c and then is discharged out of the puncture device 1c. This part of the first liquid medium brings out the air in the outer tube body 21c. It is only necessary to inject the first liquid medium into the puncture device 1c, which can simultaneously discharge the air in both the puncture needle 22c and the outer tube body 21c, thus improving the air discharging efficiency of the puncture device 1c.

Referring to Fig. 14, Fig. 17, Fig. 18 and Fig. 20, in this embodiment, the handle assembly 1c also includes an anti-miscontact member 40c. The anti-miscontact member 40c includes an anti-miscontact body 41c and a held portion 42c connected with the anti-miscontact body 41c. The anti-miscontact body 41c includes a sleeve with an opening. The anti-miscontact body 41c and the held portion 42c are integrally molded, and the held portion 42c extends away from the opening of the anti-miscontact body 41c. In other embodiments, the anti-miscontact body 41c and the held portion 42c may be different components, and they are fixedly connected together by other elements. In this embodiment, the anti-miscontact member 40c is detachably connected with the handle assembly 10c. The anti-miscontact member 40c is used for preventing the second control element 14c from moving relative to the distal end of the handle body 11c in case of misoperation. Misoperation means that the second control element 14c is touched or operated if it is not necessary to adjust the outer tube body 21c by operating the second control element 14c; or, the first control element 12c is touched or operated if it is not necessary to adjust the puncture needle 22c by operating the first control element 12c. When the anti-miscontact member 40c is connected with the handle body 11c, and the anti-miscontact body 41c sleeves the outer wall of the handle body 11c, an end face of the proximal end of the anti-miscontact body 41c is close to an end face of the distal end face of the second control element 14c. When the end face of the distal end of the second control element 14c abuts against the end face of the proximal end of the anti-miscontact member 40c, the second control element 14 cannot slide axially away from the proximal end of the handle body 11c relative to the handle body 11c, so as to prevent the second control element 14c from being mistouched in the puncture process of the puncture needle 22c and making the outer tube body 21c cover the puncture needle 22c again in advance. When the anti-miscontact member 40c is removed from the handle body 11c, the second control element 14c can slide axially away from the proximal end of the handle body 11c without the limiting effect of the anti-miscontact member 40c, so that the outer tube body 21c can also slide away from the proximal end of the handle body 11c, and the outer tube body 21c can cover the puncture needle 22c again.

In other embodiments, the handle assembly 1c may not include the anti-misoperation member 40c, as long as the purpose of preventing misoperations of the embodiments can also be achieved since the second control element 14 is abutted by other components and is not easy to slide after the second control element 14 is mistouched.

### Fifth embodiment

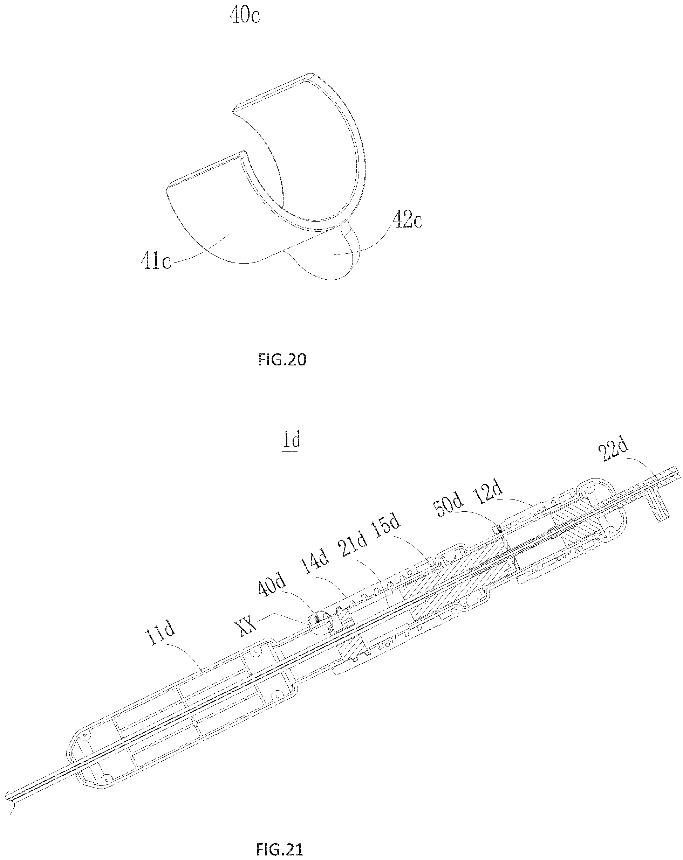

Referring to Fig. 21 to Fig. 22, the puncture device 1d provided in this embodiment is basically the same as that in the fourth embodiment. In the puncture device 1d, the second control element 14d can control the distal end of the outer tube body 21d to be bent and deformed, and the second control element 14d can also control the outer tube body 21d to axially move relative to the handle body 11d. In this embodiment, components such as the first control element 12d, the second control element 14d and the outer tube body 21d are basically the same as components such as the first control element 12c, the second control element 14c and the outer tube body 21c in the fourth embodiment, and will not be repeated here. In this embodiment, a handling member and the second control element 14d refer to the same component. In this embodiment, the most important difference from the fourth embodiment is that the anti-miscontact member 40d is different from the anti-miscontact member 40c, which is described in detail below.

The anti-miscontact member 40d includes a supporting block 41d, a stop portion 42d, and an elastic portion 43d connected between the supporting block 41d and the stop portion 42d. The second control element 14d is provided with a storage portion 140. In this embodiment, the storage portion 140 is an elastic portion receiving hole 140 that runs through inner and outer surfaces of the second control element 14d. The supporting block 41d is fixed on a side wall of the elastic portion receiving hole 140 close to the outer surface of the second control element 14d. The elastic portion 43d is received in the elastic portion receiving hole 140. The stop portion 42d is at least partially received in the elastic portion receiving hole 140, and the stop portion 42d is closer to the handle body 11d than the elastic portion 43d. An inner diameter of a portion of the elastic portion receiving hole 140 close to the inner surface is less than an outer diameter of the elastic portion 43d, so that the stop portion 42d can only be partially exposed from the elastic portion receiving hole 140 without being separated from the elastic portion receiving hole 140. Part of the outer surface of the handle body 11d is provided with an annular stop portion receiving slot 110d, and the annular stop portion receiving slot 110d surrounds the axial central axis of the handle body 11f. The stop portion receiving slot 110d can receive part of the stop portion 42d. The elastic portion 43d is supported by the supporting block 41d, and applies its own elastic force to the stop portion 42d, so that the stop portion 42d abuts against a slot wall of the stop portion receiving slot 110d. The stop portion receiving slot 110d cooperates with the stop portion 42d to restrain the axial movement of the second control element 14d. When the second control element 14d is misoperated, and an acting force of misoperation cannot separate the stop portion 42d from the stop portion receiving slot 110d, the stop portion 42d will stop the second control element 14d from moving axially relative to the handle body 11d, so that the outer tube body 21d cannot move relative to the handle body 11d. When the acting force that pushes the second control element 14d causes the stop portion 42d to retract into the elastic portion receiving hole 140, the stop portion 42d is separated from the stop portion receiving slot 110d, and the stop portion 42d cannot cooperate with the stop portion receiving slot 110d, which cannot restrain the axial movement of the second control element 14d. In this embodiment, the stop portion 42d moves away from the supporting block 41d under the action of the elastic force of the elastic portion 43d to abut against the slot wall of the stop portion receiving slot 110d. In other embodiments, the elastic portion 43d applies a pull force of the stop portion 42. The gravity of the stop portion 42d is greater than the pull force of the elastic portion 43d, so as to abut against the slot wall of the stop portion receiving slot 110d. When the acting force that pushes the second control element 14d and the pull force of the elastic portion 43d act on the stop portion 42d together to separate the stop portion 42d from the stop portion receiving slot 110d, the stop portion 42d cannot stop the second control element 14d from moving axially. Under the action of the pull force of the elastic portion 43d, the acting force that pushes the second control element 14d can be reduced.

In this embodiment, the cooperation between the anti-miscontact member 40d and the handle body 11d has the following beneficial effects. On the one hand, in the process that the first control element 12d controls the puncture needle 22d to move out of the outer tube body 21d, the second control element 14d can be prevented from being misoperated to make the outer tube body 21d cover the puncture needle 22d in advance, which affects the puncture of the puncture needle 22d. On the other hand, in the process of rotating the second control element 14d to adjust the deformation of the distal end of the outer tube body 21d, the second control element 14d can be prevented from being misoperated to drive the outer tube body 21d to move away from the proximal end of the handle body 11d, so that the outer tube body 21d moves away from the proximal end of the handle body 11d in advance, and a distance between the end face of the distal end of the outer tube body 21d and the end face of the distal end of the puncture needle 22d increases. The following phenomenon is avoided: The first control element 12d controls the puncture needle 22d to be unable to extend out of the outer tube body 21d in the direction away from the proximal end of the handle body, or the puncture needle extending out of the outer tube body 21d is too short, so that the puncture of the puncture needle 22d is affected.

In this embodiment, the second control element 14d can control the outer tube body 21d to axially move, and can also control the distal end of the outer tube body 21d to be deformed. In other embodiments, the second control element 14d can only control the outer tube body 21d to axially move, or can only control the distal end of the outer tube body 21d to be deformed.

In this embodiment, the elastic portion receiving hole 140 of the second control element 14d is arranged at its distal end portion. In other embodiments, the elastic portion receiving hole 140 can also be arranged at other positions, such as a proximal end portion, as long as the elastic portion receiving hole 140 can accommodate part of the anti-miscontact member 40d.

In this embodiment, as shown in Fig. 21, the handle assembly 10d also includes an anti-mistouch member 50d, and the structure of the anti-mistouch member 50d is basically the same as that of the anti-miscontact member 40d. The anti-mistouch member 50d is received in the first control element 12d. The anti-mistouch member 50d cooperates with the handle body 11d to prevent the first control element 12d from moving axially. The anti-mistouch member 50d is used for preventing, when the first control element 12 is mistouched, the puncture needle 22d from moving relative to the handle body 11d. The puncture surgery has the following specific beneficial effects. When the puncture device 1d is delivered to a target position, or when the second control element 14d is rotated to bend the distal end of the outer tube body 21d towards a position to be punctured (such as the fossa ovalis), the anti-mistouch member 50d restrains the axial movement of the first control element 12 to avoid the following phenomenon: The first control element 12 is mistouched to make the puncture needle 22d extend out of the outer tube body 21d and stab a patient.

In other embodiments, the anti-mistouch member 50d can be omitted, and only the anti-miscontact member 40d is provided.

In other embodiments, the stop effect of the anti-miscontact member 40d in the second control element 14d is better than that of the anti-mistouch member 50d in the first control element 12d (for example, in the anti-miscontact member 40d, the elastic force of the elastic portion 43d that enables the stop portion 42d to abut against the stop portion receiving slot 110d is greater than the elastic force of the elastic portion in the anti-mistouch member 50d that enables the stop portion to abut against the stop portion receiving slot 110d). This can further lower the risk of misoperating the second control element 14d to move the outer tube body 21d. At the same time, on the basis of preventing the first control element 12d from being misoperared, the resistance to the axial movement of the first control element 12d is reduced, so that it is convenient to push the first control element 12d to control the puncture needle 22d to move.

In other embodiments, the second control element 14d is provided with a plurality of elastic portion receiving holes 140 spaced apart from one another. The number of the anti-miscontact member 40d is the same as the number of the elastic portion receiving hole 140, so that each anti-miscontact member is received in each elastic portion receiving hole 140. The multiple anti-miscontact members 40d can enhance the miscontact prevention effect of the second control element 14d. In other embodiments, among the multiple anti-miscontact members 40d, a pair of opposite anti-miscontact members 40d are symmetrical about the central axis of the puncture device 1d. Such a setting can make the restraining effect of the multiple anti-miscontact members 40d on the second control element 14d distributed symmetrically and uniformly.

In other embodiments, the storage portion 140 can be an elastic portion receiving slot 140 formed by part of the inner surface of the second control element 14d that is outwards recessed. There is one or more elastic portion receiving slots 140, the number of which is the same as the number of the anti-miscontact member 40d. The elastic portion 43d is received in the elastic portion receiving slot 140, and the stop portion 42d is partially received in the elastic portion receiving slot 140. The elastic portion 43d is connected between a bottom wall of the elastic portion receiving slot 140 and the stop portion 42d.

In other embodiments, the storage portion 140 can be an annular slot 140 formed by part of the inner surface of the second control element 14d that is outwards recessed. The annular slot 140 is formed by part of the inner surface of the second control element 14d that is inwards recessed. One or more anti-miscontact members 40d are arranged on the annular slot 140 at intervals. The supporting block 41d is fixed on the slot wall of the annular slot 140. The elastic portion 43d is received in the annular slot 140, and the stop portion 42d is partially received in the annular slot 140. The elastic portion 43d is connected between a bottom wall of the annular slot 140 and the stop portion 42d.

In other embodiments, the anti-miscontact member 40d can only include the elastic portion 43d and the stop portion 42d, as long as the anti-miscontact member 40d can achieve limitation.

In other embodiments, the handle body 11d may not include the stop portion receiving slot 110d. The stop portion 42d abuts against the outer wall of the handle body under the action of the elastic portion 43d, and the friction force between the stop portion 42d and the handle body restrains the movement of the second control element 14d.

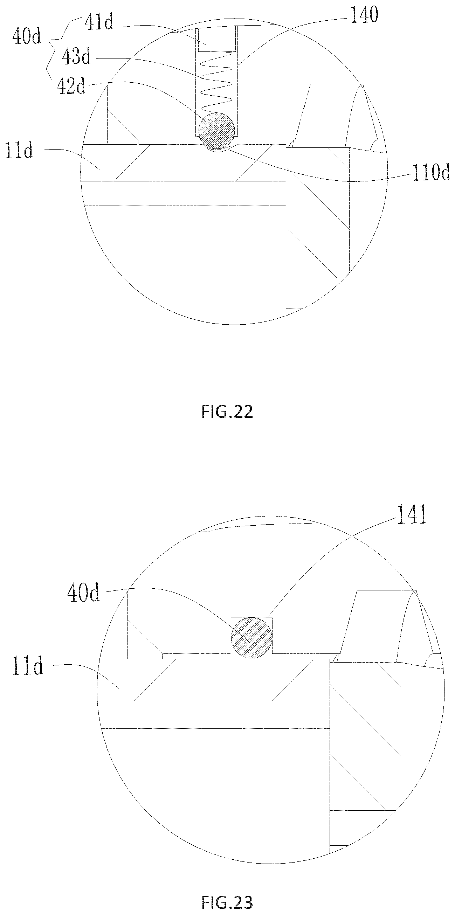

In other embodiments, as shown in Fig. 23, the anti-miscontact member 40d is a hollow tube. The anti-miscontact member 40d is elastic. A material of the anti-miscontact member 40d can be silica gel. Part of the inner surface of the second control element 14d is outwards recessed to form an annular accommodating slot 141. The anti-miscontact member 40d is fixed to the accommodating slot 141. Part of the anti-miscontact member 40d is exposed from the accommodating slot 141 and abuts against the outer wall of the handle body 11d. In the process of the axial movement of the second control element 14d, there is a friction force between the anti-miscontact member 40d and the handle body 11d, which prevents the second control element 14d from moving. When the acting force that misoperates the second control element 14d is less than a static friction force between the anti-miscontact member 40d and the handle body 11d, the second control element 14d remains stationary. An acting force on the second control element 14d makes the second control element 14d move, the friction force between the anti-miscontact member 40d and the handle body 11d plays a role of buffering the movement of the second control element 14d, which prevents the second control element 14d from moving too fast and the outer tube body 21d controlled by the second control element 14d from moving too fast.

In other embodiments, the outer tube body 21d can be integrally molded with the second fixing member 15d and the second control element 14d to form an outer tube (not shown in the figure). The anti-miscontact member 40d is connected with the second control element 14d. The anti-miscontact member 40d restrains the movement of the entire outer tube.

The features of the embodiments described above can be arbitrarily combined. In order to make the description concise, all possible combinations of various features in the above embodiments are not completely described. However, the combinations of these features should be considered as the scope described in the embodiments as long as there is no contradiction in them.

The above-mentioned embodiments only express several implementation modes of the embodiments, and their descriptions are more specific and detailed, but they cannot be understood as limiting. It should be noted that those of ordinary skill in the art can further make various transformations and improvements without departing from the concept of the embodiments, and these transformations and improvements all fall within the scope of the embodiments.

## Claims

1. A puncture device, comprising:
a handle assembly and a puncture assembly that is received in the handle assembly and fixedly connected to the handle assembly, wherein the puncture assembly comprises an outer tube and a puncture member;
the outer tube is a hollow tube; at least part of the puncture member is received in the outer tube;
a distal end of the puncture member is exposed from a distal end of the outer tube or is received in the distal end of the outer tube; and
a proximal end of the puncture member runs through a proximal end of the outer tube.

2. The puncture device according to claim 1, wherein the handle assembly further comprises a first control element which is connected with the puncture member and used for controlling the puncture member to slide.

3. The puncture device according to claim 2, wherein the handle assembly is provided with a first limiting hole, which restrains an axial travel of the first control element.

4. The puncture device according to claim 2, wherein the outer tube is slidable relative to the handle assembly.

5. The puncture device according to claim 4, wherein the handle assembly further comprises a second control element that is spaced apart from the first control element, is connected with the outer tube, and controls the outer tube to slide relative to the puncture member.

6. The puncture device according to claim 5, wherein the handle assembly is provided with a second limiting hole, which restrains an axial travel of the second control element.

7. The puncture device according to claim 5, wherein the axial travel of movement of the outer tube is greater than or equal to the axial travel of movement of the puncture member.

8. The puncture device according to claim 1, wherein the handle assembly comprises a first fixing member and a second fixing member located on a distal side of the first fixing member; the first fixing member is configured for fixing the puncture member; and the second fixing member is configured for fixing the outer tube.

9. The puncture device according to claim 8, wherein the puncture device further comprises a first connecting member; the first fixing member is provided with a first connecting member channel; and the first connecting member channel is communicated with the inside of the puncture member and the inside of the first connecting member, so that a first liquid medium is delivered into the body through the first connecting member, the first connecting member channel and the puncture member in sequence.

10. The puncture device according to claim 9, wherein the second fixing member is provided with a communicating cavity communicated with an inner cavity of the outer tube and a puncture member channel communicated with the communicating cavity; and the puncture member is threaded through the outer tube via the puncture member channel and the communicating cavity.

11. The puncture device according to claim 10, wherein the puncture device further comprises a second connecting member communicated with the communicating cavity, so that a second liquid medium is delivered into the body through the second connecting member, the communicating cavity and the outer tube in sequence.

12. The puncture device according to claim 1, wherein the puncture assembly is bendable and deformable.

13. A puncture device, comprising:
a handle assembly and a puncture assembly that is received in the handle assembly and fixedly connected to the handle assembly, wherein the puncture assembly comprises a puncture member;
the handle assembly further comprises a first control element used for controlling the puncture member; and
when the first control element rotates, the first control element controls the puncture member to move close to or away from the handle assembly.

14. The puncture device according to claim 13, wherein the first control element is connected with a proximal end portion of the puncture member, and the proximal end portion of the puncture member is received in the first control element.

15. The puncture device according to claim 14, wherein an inner surface of the first control element is provided with a first clamping portion;
the first clamping portion clamps the puncture member; and
when the first clamping portion axially moves relative to the proximal end of the handle assembly, the first clamping portion drives the puncture member to move relative to the handle assembly.

16. The puncture device according to claim 14, wherein an inner surface of the first control element is provided with a first threaded portion; the handle assembly comprises a handle body; the first control element sleeves the handle body; a proximal end portion of the puncture member is received in the handle body and connected to the first control element; and when the first control element rotates around the handle body, the puncture member is driven to move relative to the handle body.

17. The puncture device according to claim 16, wherein an inner surface of the first control element is further provided with a first clamping portion; the first threaded portion is arranged on a distal side of the first clamping portion; an outer wall of the handle body is provided with a second threaded portion used cooperatively with the first threaded portion; and part of the puncture member runs through the handle body and is clamped to the first clamping portion.

18. The puncture device according to claim 17, wherein after the first clamping portion drives the puncture member to axially move away from the proximal end of the handle assembly by a preset distance, the first control element rotates to drive the puncture member to continue to move away from the proximal end of the handle assembly.

19. The puncture device according to claim 17, wherein the handle body is provided with a first limiting hole; part of the puncture member is clamped to the first clamping portion through the first limiting hole; and an axial distance between distal ends of the first threaded portion and the second threaded portion is less than an axial length of the first limiting hole.

20. The puncture device according to claim 13, wherein the puncture member comprises a puncture needle, and a first fixing member connected to a proximal end of the puncture needle; the puncture assembly further comprises an outer tube; the outer tube comprises an outer tube body, and a second fixing member connected to a proximal end of the outer tube body; and the second fixing member is located on a distal side of the first fixing member.

21. The puncture device according to claim 20, wherein the puncture assembly further comprises a connecting member; the connecting member is a hollow tube; the first fixing member is provided with a first channel; the second fixing member is provided with a second channel; the connecting member is communicated with the inside of the first channel and the inside of the second channel, so that a first liquid medium is delivered into the body through the first channel, the connecting member, the second channel and the outer tube in sequence.

22. The puncture device according to claim 21, wherein a proximal end of the connecting member is fixed in the first channel, and a distal end of the connecting member is slidably connected into the second channel.

23. The puncture device according to claim 21, wherein the puncture assembly further comprises a sealing member, which seals the proximal side of the second channel.

24. The puncture device according to claim 16, wherein the handle assembly further comprises a second control element; the second control element is spaced apart from the first control element; the second control element is connected with the outer tube; and the second control element controls the outer tube to slide relative to the puncture member.

25. The puncture device according to claim 24, wherein the handle assembly further comprises a limiting member which is detachably connected with the handle assembly; and
when the limiting member sleeves an outer wall of the handle assembly, the limiting member abuts against the distal side of the second control element to restrain the second control element from driving the outer tube to axially move away from the proximal end of the handle assembly.

26. A puncture device, comprising:
a handle assembly and a puncture assembly that is partially received in the handle assembly and fixedly connected to the handle assembly, wherein the puncture assembly comprises an outer tube;
the handle assembly comprises an handling member and an anti-miscontact member connected with the handling member;
the handling member is connected with the outer tube; the handling member controls the outer tube to move close to or away from a proximal end of the handle assembly; and
the anti-miscontact member is used for preventing, when the handling member is misoperated, the outer tube from moving relative to the handle assembly.

27. The puncture device according to claim 26, wherein when the handling member rotates, the outer tube is bendable and deformable relative to the handle assembly.

28. The puncture device according to claim 27, wherein the handle assembly comprises a handle body; the handling member sleeves the handle body; a proximal end portion of the outer tube is received in the handle body and connected with the handling member; and when the handling member moves relative to the handle body, the outer tube is driven to move relative to the handle body.

29. The puncture device according to claim 28, wherein the anti-miscontact member is detachably connected with the handle body; and when the anti-miscontact member sleeves the handle body, a proximal side of the anti-miscontact member abuts against a distal side of the handling member.

30. The puncture device according to claim 28, wherein the anti-miscontact member is fixedly connected with the handling member.

31. The puncture device according to claim 30, wherein the anti-miscontact member comprises a stop portion, and an elastic portion connected with the stop portion; the elastic portion is received in the handling member; the stop portion is closer to the handle body than the elastic portion; and when the stop portion abuts against the handle body, the anti-miscontact member prevents, when the handling member is misoperated, the outer tube from moving relative to the handle assembly.

32. The puncture device according to claim 31, wherein part of the handling member is provided with an elastic portion receiving hole; and the elastic portion is received in the elastic portion receiving hole.

33. The puncture device according to claim 32, wherein part of an outer surface of the handle body is provided with an annular stop portion receiving slot surrounding an axial central axis of the handle body; the stop portion abuts against a slot wall of the stop portion receiving slot; and the stop portion is matched with the stop portion receiving slot to restrain the handling member from moving.

34. The puncture device according to claim 30, wherein the anti-miscontact member is received in the handling member; the anti-miscontact member abuts against the handle body; and when the handling member moves relative to the handle body, a friction force between the anti-miscontact member and the handle body prevents the handling member from moving.

35. The puncture device according to claim 26, wherein the puncture assembly further comprises a puncture member connected with the outer tube; the handle assembly further comprises a first control element; and the first control element controls the puncture member to move.

36. The puncture device according to claim 35, wherein the handle assembly further comprises an anti-mistouch member; the anti-mistouch member is received in the first control element and connected with the handle body; and the anti-mistouch member is used for preventing, when the first control element is misoperated, the puncture member from moving relative to the handle assembly.
